# EUROPÄISCHE PATENTSCHRIFT

(11) **EP 2 927 309 B1**
(45) Veröffentlichungstag und Bekanntmachung des Hinweises auf die Patenterteilung: **11.03.2020**
(21) Anmeldenummer: 15001025.4
(22) Anmeldetag: 27.07.2007
(51) Int. Cl.: C12M 1/16, C12M 3/00, C12M 1/00, C12M 1/107

(54) **BIOGASANLAGE UND VERFAHREN ZUR ERZEUGUNG VON BIOGAS AUS STROH**
BIOGAS SYSTEM AND METHOD FOR GENERATING BIOGAS FROM STRAW
INSTALLATION DE BIOGAZ ET PROCÉDÉ DE PRODUCTION DE BIOGAZ À PARTIR DE PAILLE

(30) Priorität: 27.06.2007 DE 102007029700
(43) Veröffentlichungstag der Anmeldung: 07.10.2015
(62) Teilanmeldung aus: 07786393.4
(73) Patentinhaber: Meissner, Jan A., 8700 Küsnacht (CH)
(72) Erfinder: Feldmann, Michael, 35037 Marburg (DE)
(74) Vertreter: Schüssler, Andrea

(56) Entgegenhaltungen:
- EP-A- 0 286 100
- EP-A- 0 934 998
- WO-A1-2004/016797
- WO-A2-03/043939
- DE-A1- 2 730 671
- DE-A1- 4 308 920
- DE-A1- 10 021 383
- US-A- 5 707 417
- US-A1- 2003 176 669
- US-A1- 2006 024 801
- US-A1- 2006 275 895

## Beschreibung

Die vorliegende Erfindung betrifft eine Biogasanlage und insbesondere ein Biomasse-Kraftwerk zur Biogaserzeugung nach dem Oberbegriff von Anspruch 1 und ein Verfahren zur Erzeugung von Biogas aus Strohballen.

Gegenwärtig findet Stroh in Biogasanlagen praktisch keine Anwendung als Gärsubstrat; in Biogasanlagen gelangt es lediglich indirekt in kleinen Mengen als im Festmist enthaltene Einstreu. Es besteht nämlich aus Gründen, die im Folgenden erläutert werden, ein technisches Vorurteil gegenüber der Verwendung von Stroh als Gärsubstrat.

Gegenwärtig sind in Deutschland über 3.500 Nassfermentationsanlagen im Einsatz, denen lediglich rund 20 Anlagen zur Feststoffvergärung (Trockenvergärung) von nachwachsenden Rohstoffen gegenüberstehen. Bei Nassfermentationsanlagen kommt eine Verwendung von Stroh schon deshalb nicht in Betracht, weil die nasse Gärsubstanz mit Paddelrädern bzw. Propellern umgerührt werden muss und sich das Stroh dabei um die Paddel bzw. Propeller wickeln würde. Deshalb wird in Nassfermentationsanlagen auch kaum strohenthaltender Festmist verwendet, sondern vornehmlich Gülle. Wenn man das Stroh häckseln würde, um dieses mechanische Problem zu umgehen, schwimmt das gehäckselte Stroh auf und mischt sich daher mit dem nassen Gärsubstrat nicht. Außerdem verstopft es dabei in der Regel die Ab- bzw. Überläufe der Nassfermenter. Schon aus diesen Gründen kommt Stroh als Gärsubstrat für Nassfermentationsanlagen gegenwärtig nicht in Betracht.

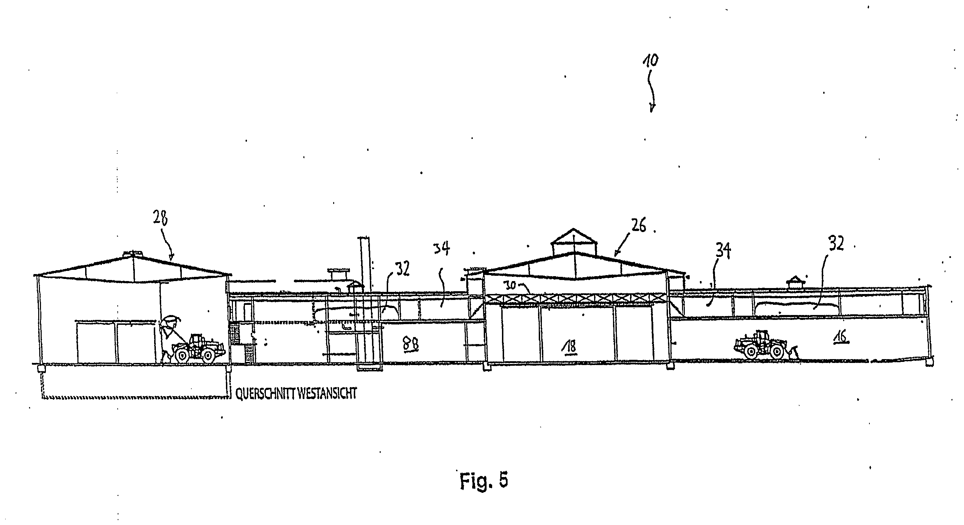

Bestehende Biomasse-Kraftwerke bzw. Biogasanlagen zur Biogaserzeugung nach dem Feststoff-Vergärungsverfahren sind typischerweise kleine landwirtschaftliche Anlagen mit zwei bis sechs Fermentern. Gemäß dem erst kürzlich erschienenen Endbericht "Monitoring zur Wirkung des novellierten Erneuerbare-Energien-Gesetzes (EEG) auf die Entwicklung der Stromerzeugung aus Biomasse", der im Auftrag des Bundesministeriums für Umwelt, Naturschutz und Reaktorsicherheit (BMU) erstellt wurde, hat die Trockenvergärung noch nicht den Stand der Marktreife erreicht. Die in Betrieb befindlichen Anlagen werden dort vielmehr als Demonstrationsanlagen angesehen (siehe Seite 52, Abb. 5-2 des genannten Endberichts).

Prinzipiell wäre es zwar möglich, in solchen Feststoffvergärungsanlagen Stroh als Gärsubstrat zu verwenden. Dies wird jedoch gegenwärtig nicht getan, weil gemeinhin angenommen wird, dass der Gasertrag von Stroh zu gering sei.

Anstelle von Stroh werden in bekannten Feststoffvergärungsverfahren stattdessen herkömmlicherweise vorwiegend Bioabfälle sowie Rinder- und Schweinefestmist und Hühnertrockenkot verwendet. Außerdem werden hauptsächlich wenig lignifizierte nachwachsende Rohstoffe (NawaRos), wie beispielsweise frisch geerntetes Gras, Silagen aus Gras, Getreide-Ganzpflanzenschnitt und Mais-Ganzpflanzenschnitt sowie Heu, Kartoffeln und Rübenhackschnitzel eingesetzt.

Da die gegenwärtig bekannten Biogasanlagen mit Feststoffvergärung typischerweise im ländlichen Raum, beispielsweise auf einem Bauernhof stehen und nur einen verhältnismäßig kleinen Durchsatz haben, wird der Bedarf an Gärmasse üblicherweise aus dem eigenen Bestand des Landwirtes und möglicherweise der Nachbarbetriebe gedeckt. Für größere Feststoffvergärungsanlagen als bisher üblich stellt die Beschaffung geeigneter Gärsubstrate in ausreichender Menge und zu wirtschaftlichen Bedingungen jedoch ein Problem dar.

EP 0 286 100 A beschreibt eine anaerobe Vergärung bei der als Einsatzstoff extrem heterogener Rohabfall eingesetzt wird, der u.a. lignozellulosehaltige Stoffe wie Papier und Pappe, aber auch biologisch nicht abbaubare Stoffe wie Kunststoffe (Folien, Tüten, Messer, Gabeln etc.), Metallgegenstände (Messer, Gabeln, Batterien, Scheibenwischermotoren, Alufolien etc.), Holz, Keramik und Glas enthält. Der Einsatz des Gärsubstrats Stroh wird jedoch nicht angegeben.

DE4308920 A1 beschreibt eine Vorrichtung zur verbesserten Vorbehandlung von Bioabfällen, insbesondere zur verbesserten Hydrolyse der biologisch abbaubaren Bioabfallfraktionen zwecks weitergehender Reduzierung des Volumens und der Masse der organischen Substanzen. Der Verwendungszweck der Vorrichtungen zur Müllbehandlung ist nicht die Optimierung des Biogas- bzw. Methanertrags, sondern die Reduzierung des zu deponierenden Müllvolumens. Auch hier wird der Einsatz des Gärsubstrats Strohs nicht angegeben.

US2006/0275895 A1 beschreibt eine nach dem Verfahren der Nassfermentation arbeitende Biogasanlage, die im Wesentlichen aus einem Häcksler, einem dem Fermentationsreaktor vorgeschalteten Anmischtank, einem Fermentationsreaktor, einem ersten Separator, einem zweiten Separator und einer nachgeschalteten Hydrolyseeinheit besteht. Als Einsatzstoffe werden alle typischen Gärsubstrate wie organischen Abfall, Klärschlamm, Mais, Gras, natives Stroh, Getreideganzpflanzensilage, Maissilage, Heu und Wirtschaftsdünger (Kot, Urin, Gülle, strohhaltiger Festmist) verwendet.

WO2004/016797 A1 beschreibt ein Verfahren und eine Anlage zur Erzeugung von Biogas aus organischem Material. Diese besteht im Wesentlichen aus einer Standard- bzw. Getreidekornmühle (ggf. auch nur aus einer einfachen Mühle für die Zerreibung von Grünmassepellets), einem Anmischtank mit einem Rührwerk, einem Fermentationsreaktor mit einem Rührwerk, Pumpen und einer Steuereinheit. Als Einsatzstoff kann jede Art von Grünmasse dienen sowie Schlachtabfälle und Gülle. Ohne auf die unterschiedlichen spezifischen Eigenschaften der Einsatzstoffe einzugehen, versteht diese Anmeldung unter Grünmasse alle Pflanzen, die zur Erzeugung der Pflanzenmasse den natürlichen Prozess der Photosynthese nutzen wie z.B. Silagen, Stroh, Getreide, Spreu, Raps, Zuckerrüben, Steckrüben, Mais, Sonnenblumen, Kohl, Kartoffeln, Molasse, Erbsen, Bohnen, Linsen, Flachs, Lupinen, Weidepflanzen wie Luzerne, Gras und Klee, Rasengrün, Straßenbegleitgrün, Heu und Blätter von Bäumen. Ferner können als Ko-Substrate Gülle und Schlachtabfälle verwendet werden.

EP 0 934 998 A1 liegt die Aufgabe zugrunde, ein Verfahren zur Methanisierung von Biomassen sowie eine zur Durchführung des Verfahrens geeignete Vorrichtung zu schaffen, bei der mit minimalem apparativem Aufwand und bei einfachster Verfahrensführung ohne Pumpen des Substrats eine Methanisierung auch in kleinen Chargen durchgeführt werden kann. Zur Lösung dieser Aufgabe ist vorgesehen, dass der in einem Behälter angeordneten, von einer luftundurchlässigen Hülle umgebenen Biomasse ein Impfmaterial als Einmalzugabe zugesetzt und die so gebildete Reaktionsmasse ohne weiteres Nachimpfen unter Luftabschluss vergoren wird. Außer im Fall der erstmaligen Inbetriebnahme besteht das Impfmaterial für die weiteren Batch-Ansätze aus den Gärresten, die nach dem Ende eines Methanisierungsdurchlaufs anfallen.

US2006/0275895A1 beschreibt eine Biogasanlage, die im Wesentlichen aus einem Häcksler, einem dem Fermentationsreaktor vorgeschalteten Anmischtank, einem Fermentationsreaktor, einem ersten Separator, einem zweiten Separator und einer nachgeschalteten Hydrolyseeinheit besteht. Als Einsatzstoffe werden alle typischen Gärsubstrate wie organischer Abfall, Klärschlamm, Mais, Gras, natives Stroh, Getreideganzpflanzensilage, Maissilage, Heu und Wirtschaftsdünger (Tier-Kot, Tier-Urin, Gülle, strohhaltigen Festmist) verwendet. Das von US2006/0275895A1 beschriebene Verfahren ist ein Nassverfahren, das in einer Nassanlage durchgeführt wird.

WO 03/043939 A2 betrifft die Behandlung von Müllfraktionen aus dem Fachgebiet der Müllentsorgung, bei der es vornehmlich darauf ankommt, das Müllvolumen zu reduzieren, insbesondere im Lichte des gesetzlichen Verbotes der Mülldeponierung. Als Einsatzstoffe dienen die Müllfraktionen Essensreste, Klärschlamm, Bioabfall und Gartenabfälle.

US 5,707,417 betrifft die Behandlung von Biomüll aus dem Fachgebiet der Müllentsorgung, bei der es vornehmlich darauf ankommt, das Müllvolumen zu reduzieren. Als Einsatzstoffe dienen vornehmlich Essensreste wie Kohl, gekochter Reis, Sardinen, Butter und Muscheln, die in wässrige Suspension mit einem Wasseranteil von 88,7% und einem Trockensubstanzanteil von 11,3% überführt werden. Die flüssige Biomüll-Suspension wird in einen Vorratstank eingeführt und dort bis zur Verwendung zwischengelagert. Von dort aus wird sie über eine Leitung in einen Autoklaven eingebracht und dort in Anwesenheit von Wasser bei einer Temperatur von 100°C - 400°C einem Druck größer als Sattdampfdruck ausgesetzt, um zumindest einen Teil der zuvor ungelösten Komponenten des Biomülls in wässrige Lösung zu überführen. Die wässrige Suspension mit den gelösten organischen Komponenten wird einer Fest-Flüssig-Trennung unterzogen und die flüssige Phase einer anaeroben Vergärung zugeführt zur Erzeugung von Biogas.

Der vorliegenden Erfindung liegen die Ideen für eine Biogasanlage, insbesondere für ein Biomasse-Kraftwerk und für ein Verfahren zur Erzeugung von Biogas zugrunde, die das Problem lösen, geeignete Gärsubstrate in ausreichenden Mengen insbesondere für größere, im industriellen Maßstab arbeitende Anlagen zu wirtschaftlichen Bedingungen zu beschaffen und zu verarbeiten.

Diese Ideen werden mit einer Biogasanlage mit den Merkmalen des Anspruchs 1 und mit dem Verfahren mit den Merkmalen des Anspruchs 14 realisiert. Vorteilhafte Weiterbildungen sind in den abhängigen Ansprüchen angeben.

Erfindungsgemäß ist bei der Biogasanlage eine Einrichtung zum thermischen Aufschluss von Strohballen vorgesehen. Durch den Aufschluss von Stroh lässt sich entgegen der üblichen Meinung auch mit diesen ein erheblicher Gasertrag erzielen. Dadurch wird die Biogaserzeugung, insbesondere die Biogaserzeugung nach dem Feststoffvergärungsverfahren, für eine neue Klasse nachwachsender Rohstoffe zugänglich, denn Stroh steht in großen Mengen zur Verfügung steht und ist preisgünstig erhältlich. Das Stroh kann als Ergänzung zur herkömmlichen Frischmasse, wie beispielsweise Silage aus Grünschnitt, Silage aus ganzen Getreidepflanzen etc., verwendet werden und stellt so die Versorgung selbst einer Vielzahl großer und sehr großer Biomasse-Kraftwerke, mit beispielsweise 20 oder mehr Fermentern und einer jeweiligen elektrischen Leistung von über 5.000 kW zu wirtschaftlichen Bedingungen sicher.

Vorzugsweise umfasst die Einrichtung zum thermischen Aufschluss von Stroh eine Einrichtung zur Sattdampfbehandlung. Die Einrichtung zur Sattdampfbehandlung umfasst vorzugsweise einen Druckbehälter und Mittel, die geeignet sind, im Druckbehälter einen Wasserdampf zu erzeugen, mit einem Druck, der zwischen 20 und 30 bar liegt und einer Temperatur, die zwischen 180°C und 250°C liegt. Eine Sattdampfbehandlung findet bei den beschriebenen Drücken und Temperaturen statt und dauert typischerweise 5 bis 15 Minuten. Die Funktion der Sattdampfbehandlung soll am Beispiel von Weizenstroh beschrieben werden.

Weizenstroh besteht zu etwa 40 % aus Cellulose, zu 23 % aus Arabinoxylan (Hemicellulose) und zu 21 % aus Lignin, wobei alle drei Hauptkomponenten eine dichtgepackte Struktur einnehmen. Das wesentliche Hindernis für die biochemische Verwertung der Cellulose und der Hemicellulose ist das für Mikroorganismen unverdauliche Lignin, das den Bakterien den Weg zur Cellulose und zur Hemicellulose versperrt. Bei der Sattdampfbehandlung werden die Ligninstrukturen aufgeweicht bzw. geschmolzen, aber während der verhältnismäßig kurzen Behandlungsdauer im Wesentlichen nicht aus den Halmen ausgelöst. Nach der Sattdampfbehandlung erstarrt das Lignin wieder. Beim Erstarren des Lignins bilden sich jedoch aufgelockerte tröpfchenartige Ligninstrukturen, die ausreichend Zwischenräume lassen, durch die zunächst wässrigen organischen Säuren und dann die Bakterien an die Cellulose und das Arabinoxylan gelangen können, die diese durch die bekannte anaerobe vierstufige Vergärung zersetzen.

Bei der hier beschriebenen Sattdampfbehandlung wird das Lignin also vornehmlich in seiner mikroskopischen Struktur verändert, es wird aber nicht aus den Strohhalmen herausgelöst. Insbesondere bleibt die Struktur der Strohhalme als solche dabei erhalten. Dies stellt einen Unterschied zur Thermodruckhydrolyse dar, die unter grundsätzlich ähnlichen Bedingungen, jedoch für längere Zeiträume durchgeführt wird, und bei der eine echte Hydrolyse stattfindet, also eine Auflösung vormals fester bzw. trockener Stoffe in Wasser. Durch eine Thermodruckhydrolyse wird die Struktur der Strohhalme aufgelöst, und es ergibt sich eine sirupartige Suspension.

In einer vorteilhaften Weiterbildung umfasst die Einrichtung zum Aufschluss von Stroh einen Behälter zum Einweichen desselben vor der Sattdampfbehandlung, beispielsweise in Wasser. Wenn das eingeweichte Stroh einer Sattdampfbehandlung unterzogen wird, verdampft das eingezogene Wasser schlagartig, wodurch Ligno-Cellulosestrukturen zerreißen und die Cellulose noch besser für die Bakterien zugänglich wird.

Hierin beschrieben ist eine Einrichtung zum mechanischen Zerkleinern des Strohs vorgesehen, durch die das Stroh vor der Sattdampfbehandlung mechanisch aufgeschlossen werden kann, beispielsweise durch Häckseln. Dies trägt weiter zum Auflösen der Ligninstrukturen bei und erleichtert die nachfolgende Vergärung.

Wie hierin beschrieben findet eine Vermahlung des Strohs, vorzugsweise in einer Hammermühle, statt. Diese Aufschlussform zerstört die Ligninstrukturen mechanisch. Eine Vermahlung allein ist jedoch bei der Nutzung des Feststoffvergärungsverfahrens nicht sinnvoll, weil sich sonst ein teigartiger Brei bilden würde, der die Gärmasse verklebt und eine Perkolation verhindert.

Erfindungsgemäß wird das Stroh in Ballenform aufgeschlossen , was insbesondere dessen Transport und Handhabung wesentlich erleichtert, wie unten näher erläutert wird. Da die Struktur beispielsweise von Strohhalmen unter der Sattdampfbehandlung erhalten bleibt, behalten auch Strohballen ihre Form unter der Sattdampfbehandlung bei und können nach dieser einfach und effizient transportiert werden. Ein besonderer Vorteil von Ballen besteht ferner darin, dass diese zuunterst in einen Garagenfermenter geschichtet werden können, wodurch sich die Füllhöhe des Fermenters steigern läßt. Prinzipiell ist die Füllhöhe des Fermenters dadurch begrenzt, dass ab einer gewissen Höhe des Substrats im Fermenter der Druck an dessen Boden so hoch wird, dass das Substrat zu stark verdichtet wird, um Perkolat durchsickern zu lassen. Strohballenschichten, die zuunterst in einen Fermenter eingebracht werden, sind jedoch weitaus druckstabiler als herkömmliches Gärsubstrat. Selbst unter hohem Druck ist die Strohballenschicht noch durchlässig für Perkolat, so dass die übliche Füllhöhe im Fermenter noch auf die Strohballenschicht aufgefüllt werden kann. Die Fermenter können deshalb um die Höhe der Strohballenlage höher konstruiert werden als üblich, was die fermenterspezifischen Technikkosten (Tor, Gastechnik, Sensorik, Klappen und Öffnungen, Perkolatdüsen, Abläufe, Verrohrung, Pumpen etc.) konstant hält und die Effizienz der Biogasanlage als Ganzes erhöht.

Um die Effizienz einer Einweichphase und/oder einer Sattdampfbehandlung von Strohballen zu erhöhen, sind in einer vorteilhaften Weiterbildung Mittel zum Perforieren der Ballen vorgesehen. Dabei sind die Mittel zum Perforieren vorzugsweise geeignet, einen Ballen von zwei Seiten so zu perforieren, dass die bei der Perforation von einer Seite entstehenden Löcher und die bei der Perforation von der anderen Seite entstehenden Löcher durch Materialbrücken getrennt sind. Durch eine derartige Perforierung des Ballens werden sowohl das Einweichen des Ballens als auch eine nachfolgende Sattdampfbehandlung effizienter.

In einer vorteilhaften Weiterbildung umfasst die Einrichtung zur Sattdampfbehandlung mindestens einen Spieß, auf den ein Strohballlen aufspießbar ist, wobei der Spieß einen inneren Hohlraum aufweist, in den Wasserdampf einleitbar ist, und eine Mehrzahl von Öffnungen umfasst, durch die der Wasserdampf aus dem Hohlraum austreten kann. Dadurch kann der unter der Sattdampfbehandlung verwendete heiße und unter hohem Druck befindliche Wasserdampf durch den Spieß in den Ballen eingeführt werden, wodurch erreicht wird, dass die Sattdampf-Atmosphäre auch das Material im Inneren des Ballens in auszeichnendem Maße erreicht. Bei einer einfacheren Version, bei der die Sattdampf-Atmosphäre auf naheliegende Weise in den Druckbehälter eingeführt würde, kann das Problem auftauchen, dass die in dem Ballen vorhandene Luft in einem inneren Abschnitt des Ballens komprimiert wird, sich aber mit dem Dampf nicht schnell genug durchmischt, so dass die Sattdampfbehandlung in diesem inneren Abschnitt des Ballens weniger wirksam verläuft.

Falls, wie hierin beschrieben, loses Stroh verwendet wird, ist in dem Druckbehälter ein für Wasserdampf durchlässiger Behälter zur Aufbewahrung derselben vorgesehen. Ferner sind Mittel zum Transportieren des für Wasserdampf durchlässigen Behälters in und aus dem Druckbehälter vorgesehen, beispielsweise Schienen oder eine Rollenbahn.

Wie hierin beschrieben, hat der Druckbehälter eine obere Öffnung, durch die er mit losem Stroh beschickbar ist, und eine untere Öffnung, durch die das lose Stroh aus dem für Wasserdampf durchlässigen Behälter herausfallen kann. Wie unten näher erläutert wird, kann durch solch einen Aufbau eine "quasi kontinuierliche" Sattdampfbehandlung durchgeführt werden, bei der das lose Stroh chargenweise in den Behälter eingeschüttet wird, der Druckbehälter dann für die Sattdampfbehandlung geschlossen wird, danach das behandelte lose Material durch die untere Öffnung aus dem Behälter fallengelassen wird und schließlich eine nachfolgende Charge durch die obere Öffnung in den Behälter fallengelassen wird.

In einer vorteilhaften Weiterbildung kann die Einrichtung zur Sattdampfbehandlung mehrere Druckbehälter umfassen, die durch Rohrleitungen miteinander verbunden sind. Auf diese Weise kann eine Mehrzahl von Druckbehältern durch eine einzige Dampfquelle, beispielsweise ein einziges Dampfreservoir, versorgt werden, was die Effizienz insbesondere bei größeren Kraftwerken deutlich erhöht.

In einer vorteilhaften Weiterbildung umfasst die Einrichtung zum Aufschluss von Stroh einen Behälter zum Einweichen desselben in einer Wasser-Laugen-Lösung, einer Wasser-Säure-Lösung, Perkolat oder Gülle. Dieses Einweichen stellt ein Beispiel für den eingangs genannten chemischen Aufschluss dar. Dieses Einweichen kann insbesondere nach der Sattdampfbehandlung durchgeführt werden, und zwar sowohl bei hierin lediglich beschriebenem losem Stroh wie auch bei erfindungsgemäßem ballenförmigem Stroh, wobei die Ballen in diesem Fall vorzugsweise auf die oben beschriebene Weise perforiert sind. Durch dieses Einweichen wird eine (schwach aerobe) Vorhydrolyse initiiert, die vor dem Einbringen in den Fermenter stattfindet. Dazu kann das eingeweichte Stroh nach der Einweichung und vor dem Einbringen in den Fermenter vorzugsweise noch auf 30 bis 50° erwärmt werden. Diese Erwärmung kann mit dem Transport des Strohs von der Einrichtung zum Aufschluss zum Fermenter kombiniert werden, wie unten näher erläutert wird. Durch diese Vorhydrolyse beschleunigt sich die anschließende anaerobe bakterielle Fermentation im Fermenter nochmals.

Man beachte, dass der Aufbau der Biogasanlage und das Verfahren zur Erzeugung von Biogas mit den hier beschriebenen Aufschlussverfahren ohne die Zugabe von zusätzlichen Hefen, Pilzen oder Enzymen auskommt. Tatsächlich wird das ligninhaltige Material allein der Autohydrolyse und der bakteriellen Hydrolyse überlassen. Die Verwendung von Bakterien statt Hefen, Pilzen oder Enzymen ist wesentlich wirtschaftlicher, da die chemischen und biochemischen Prozesse bei der bakteriellen Hydrolyse schneller ablaufen als bei der enzymatischen. Außerdem werden gegenüber dem externen Einsatz von Enzymen und/oder Säuren erhebliche Kosten für deren Beschaffung und Handhabung eingespart.

Eine wichtige Weiterbildung der Erfindung besteht in der Weise, wie der zusätzliche Verfahrensschritt des Strohaufschlusses in den Betrieb der Biogasanlage integriert wird. Die Einrichtung zum Aufschluss des Strohs lohnt sich wirtschaftlich vor allem dann, wenn der Durchsatz der Anlage, bzw. des Biomasse-Kraftwerks hoch ist. Gegenwärtig bekannte Biomasse-Kraftwerke zur Feststoffvergärung sind jedoch im Gegenteil meistens sehr klein und auf den ländlichen Raum beschränkt. Sie verfügen über zwei bis sechs kleinere Fermenter und erreichen eine effektive elektrische Leistung von lediglich 100 bis 700 kW. Dies liegt zum einen daran, dass die Trockenfermentation gemeinhin als noch nicht marktreif angesehen wird, zum andern aber auch an der gestuften Mindestvergütung des EEG für eingespeisten Strom aus NawaRo-Anlagen, die bei der Überschreitung einer Grenze von 500 kW um bis zu 15 % abfällt. Außerdem spricht derzeit gegen eine größere Auslegung von Biogasanlagen mit Trockenfermentation, dass der erforderliche Nachschub an Gärsubstrat gemäß den Anforderungen der Financiers über Jahre im Voraus gesichert sein muss, und dass sich die Betreiber, bei denen es sich typischerweise um Landwirte handelt, auf die von ihnen selbst erzeugbaren NawaRos verlassen wollen.

Durch die erfindungsgemäße Biogasanlage und das erfindungsgemäße Verfahren, die die Verwendung von Strohballen gestatten, kann die Versorgung mit Gärsubstrat jedoch auch für weit größere Anlagen sichergestellt werden, da beispielsweise Stroh beim Getreideanbau in weit größeren Mengen anfällt, als es derzeit benötigt wird, und da es sich mit entsprechender (Groß-)Technik auch über größere Strecken verhältnismäßig wirtschaftlich transportieren lässt. Andererseits lohnen sich die Investitions- und Betriebskosten für eine Einrichtung zum Aufschließen des Strohs umso mehr, je größer der Durchsatz der Biogasanlage ist. Ein inhaltlicher Zusammenhang zwischen der Möglichkeit zum Aufschluss des Strohs und der Größe der Biogasanlage besteht insofern, als die Einrichtung zum Aufschluss des Strohs wesentlich dazu beiträgt, die Versorgung auch größerer Biogasanlagen mit Gärsubstrat sicherzustellen, und andererseits die Größe der Biogasanlage und die Verwendung der preisgünstigen Substratart Stroh die Investition für die Einrichtung zum Aufschluss im Besonderen und das große Biomasse-Kraftwerk als Ganzes erst wirtschaftlich macht.

Bei einer bisher nicht bekannten Größe von Biogasanlagen mit beispielsweise 15 bis 30 großen Garagenfermentern muss der Betrieb der Biogasanlage, und insbesondere der Transport des Gärsubstrats und der Gärreste effizient gestaltet werden. Eine weitere Aufgabe besteht darin, den oben beschriebenen Aufschluss des Strohs in den Betriebsablauf der Biogasanlage zu integrieren.

In einer vorteilhaften Weiterbildung sind die Einrichtungen zum Aufschluss von Strohballen in einem Anliefer- und Verladebereich der Biogasanlage untergebracht. Der Anliefer- und Verladebereich umfasst vorzugsweise stationäre Fördertechnik, die geeignet ist, Frischmasse aus dem Anliefer- und Verladebereich zu einem Fermentervorplatz zu fordern, von dem aus eine Mehrzahl von Fermentern des Garagentyps zugänglich ist. Während bei herkömmlichen Biogasanlagen die Frischmasse und die Gärreste mit einem Radlader transportiert werden, ist nach dieser Weiterbildung stationäre Fördertechnik vorgesehen, durch die sich auch große Mengen an Frischmasse effizient zum Fermentervorplatz transportieren lassen, um von dort in die Fermenter eingebracht zu werden. Auch gestattet eine derartige stationäre Fördertechnik es, die gesamte Biogasanlage einzuhausen, wodurch eine Geruchsbelästigung der Umwelt vermieden wird und es möglich wird, die Biogasanlage auch in der Nähe von Wohngebieten zu betreiben.

Wenn die Biogasanlage komplett eingehaust ist, stellt der Anliefer- und Verladebereich gewissermaßen eine Schnittstelle zwischen dem eingehausten Innenbereich der Anlage und dem Außenbereich dar, und ist somit in einem äußeren Abschnitt der Anlage angeordnet. Der Fennentervorplatz hingegen ist aus logistischen Gründen zentral in der Anlage angeordnet. Durch die stationäre Fördertechnik kann die Frischmasse bzw. das Gärsubstrat vom Anliefer- und Verladebereich zum Fermentervorplatz gebracht werden, ohne dass Transportfahrzeuge dazu benötigt würden, die Abgas innerhalb des Einhausungsbereichs erzeugen würden und die außerdem die Betriebskosten erhöhen würden. Wie hier beschrieben, herrscht im Anliefer- und Verladebereich ein leichter Unterdruck, so dass auch beim Anliefern von Frischmasse und beim Verladen von Gärresten nur wenig Luft nach außen dringt, und somit die Geruchsbelästigung minimal ist.

Wie beschrieben, befindet sich in dem Anliefer- und Verladebereich mindestens ein eingehauster Anlieferbunker für Frischmasse. Ferner sind vorzugsweise erste Fördermittel vorgesehen, die geeignet sind, Frischmasse aus dem mindestens einen Anlieferbunker für Frischmasse zu einem Frischmassebunker zu befördern. Diese ersten Fördermittel können beispielsweise Förderschnecken, Elevatoren und Förderbänder umfassen, auf denen die Frischmasse aus verschiedenen Anlieferbunkem zum Frischmassebunker befördert werden. Dies hat den Vorteil, dass die Frischmasse bereits dadurch, dass sie aus unterschiedlichen Anlieferbunkern auf ein und denselben Haufen gegeben wird, durchmischt wird, so dass ein späteres Durchmischen der Frischmasse sich erübrigt, bzw. nicht mehr so intensiv vorgenommen werden muss. Bei dieser beschriebenen Frischmasse handelt es sich nicht um das aufzuschließende Stroh, sondern um zusätzliche Frischmasse, wie sie in bisher bekannten Biogasanlagen mit Feststoffvergärung verwendet werden.

Ferner umfasst der Anliefer- und Verladebereich zweite Fördermittel, insbesondere ein Schubschild, welche geeignet sind, die Frischmasse durch den Frischmassebunker hindurch in Richtung auf den Fermentervorplatz zu befördern. Der Frischmassebunker hat dabei eine zweifache Funktion: zum einen dient er als Transportweg vom Anlieferbereich zum Fermentervorplatz, zum anderen dient er als Zwischenspeicher für Frischmasse. Wichtig ist dabei, dass die Frischmasse, die als erstes in den Frischmassebunker eingebracht wurde, diesen auch als erstes verlässt. Das bedeutet, dass die Frischmasse, die auf den Fermentervorplatz geliefert wird, stets etwa gleich alt und damit gleich stark vorhydrolisiert ist. Damit ergibt sich eine Substrat-Konstanz, die für die anschließende Vergärung vorteilhaft ist.

Es umfasst der Anliefer- und Verladebereich in einer vorteilhaften Weiterbildung eine Entladungsstelle für Stroh in Ballenform. In der Entladungsstelle ist vorzugsweise ein Kran vorgesehen, der geeignet ist, Ballenmaterial zu greifen und zu befördern.

In dem Anliefer- und Verladebereich ist im Übrigen wie oben erwähnt die Einrichtungen zum Aufschluss von Strohballen vorgesehen, die von einer der eingangs beschriebenen Arten ist. Insbesondere ist die Einrichtung zum Aufschluss wie oben beschrieben so ausgebildet, dass die Ballenform erhalten bleibt, so dass das durch Aufschluss vorbehandelte Stroh in Ballenform von dem Anliefer- und Verladebereich zum Fermentervorplatz transportiert werden kann, was den Transport und die Einbringung in die Fermenter sehr effizient macht.

Vorzugsweise sind dabei dritte Fördermittel, insbesondere Rollenförderer oder Schubförderer vorgesehen, die geeignet sind, einzelne Ballen oder Pakete von Ballen entlang eines Ballen-kanals zum Fermentervorplatz zu befördern.

Das Ballenmaterial wird durch die dritten Fördermittel und den Ballen-Kanal auf sehr effiziente Weise von der Peripherie zum Fermentervorplatz transportiert, was einen hohen Durchsatz bei sehr geringen Betriebskosten ermöglicht. Vorzugsweise ist an dem Fermentervorplatz nahen Ende des Ballen-Kanals ein Umsetzer angeordnet, der geeignet ist, Pakete von Ballen aus dem Ballen-Kanal zu entnehmen und an einen Radlader oder Gabelstapler als Paket zu übergeben. Wie unten näher erläutert wird, ist es vorteilhaft, in einem jeden Fermenter zuunterst eine Schicht aus Ballenmaterial einzubringen. Dies lässt sich nach dieser Weiterbildung der Erfindung wiederum besonders effizient und schnell erledigen, wenn bereits geeignete Pakete von Ballen, beispielsweise Pakete aus acht Ballen, an den Radlader oder Gabelstapler übergeben werden, die dann so, wie sie sind, im Fermenter abgeladen werden können.

Wie beschrieben, sind der Anlieferbunker, der Frischmassebunker und/oder der Ballenkanal beheizbar, mittels Abwärme, welche von einem oder mehreren Gasmotoren erzeugt wird. Durch die Vorerwärmung der Frischmasse werden Temperaturverluste ausgeglichen, die bei der Gärmassenauffrischung am Altmaterial entstehen. Dadurch wird das Wiedereinsetzen der Biogasbildung nach der Gärmassenauffrischung beschleunigt. Ferner wird dadurch die oben beschriebene schwach aerobe Vorhydrolyse möglich, die die Zeit bis zum vollständigen Ausgären der Gärmasse verkürzt und die Anlagenleistung (den Substratdurchsatz) und damit die Wirtschaftlichkeit erhöht.

In einer vorteilhaften Weiterbildung ist ein Gärrestebunker vorgesehen, der vom Fermentervorplatz aus zum Einbringen von Gärresten zugänglich ist. Der Gärrestebunker enthält wie hier beschrieben stationäre Fördermittel, die geeignet sind, Gärreste durch den Gärrestebunker hindurch abzutransportieren. Wie beschrieben, werden diese stationären Fördermittel durch Förderschnecken gebildet, die an den Enden des Gärrestebunkers angeordnet sind. Der Gärrestebunker ist vorzugsweise so bemessen, dass er das zu erwartende Aufkommen an Gärresten von mindestens zwei Tagen fasst.

Der Gärrestebunker hat eine dreifache Funktion. Erstens dient er als Zwischenspeicher für Gärreste, und zweitens stellt er die Transportvorrichtung für Gärreste aus dem zentralen Fermentervorplatz zur Peripherie bereit. Durch die ausreichende Größe des Gärrestebunkers wird erreicht, dass die Gärreste mindestens zwei Tage zwischengelagert werden können, so dass sie nicht an Wochenenden abgeholt werden müssen, an denen der Lastwagenverkehr eingeschränkt ist. Schließlich findet in dem Gärrestebunker als dritte Funktion eine Nachvergärung statt, und deshalb ist er an das Biogassystem angeschlossen. So wird aus den Gärresten weiteres Biogas gewonnen, welches bei einer einfacheren Konstruktion verloren wäre.

Am Eintrittsende des Gärrestebunkers ist vorzugsweise ein Schütttrog für Gärreste angeordnet. Die Gärreste können somit direkt vom Fermentervorplatz in den Schütttrog geschüttet werden; sie werden danach automatisch zur Peripherie transportiert.

Vorzugsweise ist am Austrittsende des Gärrestebunkers eine Einrichtung zum Dehydrieren der Gärreste vorgesehen. In den Gärresten befindet sich nährstoff- und bakterienreiches Perkolat, welches über eine Ringleitung in die weiter unten beschriebenen Perkolatumlauftanks eingebracht werden kann, wenn dafür Bedarf besteht. Falls solches Perkolat benötigt wird, wird es an der Einrichtung zum Dehydrieren aus den Gärresten ausgepresst und über die Ringleitung den Perkolatumlauftanks zugeführt. Andernfalls kann die Dehydriereinrichtung umgangen werden, und die nasseren Gärreste werden so, wie sie sind, abtransportiert.

Zusätzlich oder alternativ kann eine Trocknungsanlage zum Trocknen von Gärresten vorgesehen sein, die vorzugsweise Abwärme eines Gas-Ottomotors zum Trocknen der Gärreste verwendet. Ferner ist vorzugsweise eine Vergasungsanlage vorgesehen, die geeignet ist, aus getrockneten Gärresten nach dem Verfahren der Holzvergasung, insbesondere basierend auf Verschwelung oder Pyrolyse, Holz- bzw. Schwachgas zu erzeugen. Dieses Schwachgas kann dann dem durch die Vergärung erzeugten Biogas zugefügt werden. Durch die nachgeschaltete Vergasung der Gärreste lassen sich noch einmal rund 20% des Aufkommens an Biogas als Holz-/Schwachgas erzeugen, wodurch die Effizienz des Rohstoffs für die Gaserzeugung wesentlich erhöht wird. Insbesondere besteht ein technischer Zusammenhang zwischen der Verwendung von Stroh mit Voraufschluss und der nachgeschalteten Holzvergasung. Zum einen ermöglicht die Vergasung der Gärreste den Gasertrag zu erhöhen, wenn dieser durch einen unvollkommenen Aufschluss des Strohs geringer ausfallen sollte, als biologisch möglich wäre. Außerdem eignen sich gerade ligninhaltige Stoffe, wie der Name bereits sagt, für die Holzvergasung. Insofern ergänzen sich die Verwendung von Stroh als Gärsubstrat und die nachgeordnete Holzvergasung von Gärresten auf ideale Weise.

Die obigen Weiterbildungen der Erfindung sind auf Anlagen zur Feststoffvergärung bezogen.

Wie hierin beschrieben kann Stroh durch Vermahlen mechanisch aufgeschlossen werden, wodurch die Ligninstrukturen ebenfalls aufgerissen werden. Wenn beispielsweise vermahlenes Stroh in eine Nassfermentationsanlage eingeführt würde, würde sich nach Untersuchungen des Erfinders entgegen der in der Fachwelt verbreiteten Ansicht daraus ein erheblicher zusätzlicher Gasertrag ergeben. Durch die Vermahlung werden die flächigen Ligninstrukturen nämlich ebenfalls zerstört. Die Cellulose und das Arabinoxylan können dann durch die in der Gärmasse enthaltenen wässrigen organischen Säuren aufgelöst werden, welche auch in der in Nassfermentationsanlagen typischerweise vorhandenen Gülle und in noch stärkerem Maße in reinen NawaRo-Nassanlagen, die ohne Gülle arbeiten, enthalten sind. Dadurch wird bislang als untauglich erachtete Biomasse für die anaeroben methanerzeugenden Bakterien zugänglich.

Ein ähnlich einfaches Verfahren zum chemischen Aufschluss ist auch in Biogasanlagen zur Feststoffvergärung möglich. Dabei wird Stroh bereits Tage vor der Einbringung in den Fermenter mit anderem Frischmaterial, vorzugsweise mit Festmist vermischt. Der im Festmist vorhandene Harnstoff kann dann wiederum das Lignin anlösen und die Cellulose und das Arabinoxylan für die Hydrolyse zugänglich machen. Wichtig ist hierbei, dass zusätzlich bzw. separat bereitgestellte lignifizierte nachwachsende Rohstoffe (LHNawaRo) durch den im Festmist enthaltenen Harnstoff chemisch aufgeschlossen werden. Die Vermischung des losen ligninhaltigen Materials mit dem Festmist würde dabei typischerweise mit größerem zeitlichen Abstand vor der Einbringung in den Fermenter stattfinden, vorzugsweise einige Tage. Wie hierin beschrieben, können Schichten aus Festmist und Schichten aus ligninhaltigem Material ohne zeitlichen Vorlauf abwechselnd im Fermenter aufgebaut werden, wobei gegebenenfalls auch Schichten aus anderen, nicht stark lignifizierten NawaRos dazwischen liegen können. Dadurch kann der Harnstoff der oben liegenden Festmistschicht mit dem Perkolat in die Schicht mit dem ligninhaltigen Material eindringen und die flächigen Ligninstrukturen zumindest teilweise auflösen. Auch ist es möglich, loses ligninhaltiges Material mit den Gärresten und gegebenenfalls weiteren NawaRos zu vermischen. Dabei sorgt das saure Perkolat dafür, dass sich die flächigen Ligninstrukturen zumindest teilweise auflösen und das Material Biogas liefert, wenn auch nicht mit dem Ertrag, der sich durch die anderen oben beschriebenen Verfahren zum Aufschluss, insbesondere der Sattdampfbehandlung, erzielen lassen.

Wie beschrieben, wird das Animpfmaterial, das zusammen mit der Frischmasse wieder in den Fermenter kommt, vor der Vermischung mittels einer mechanischen Presse, wie beispielsweise einer Schneckenpresse, durchgewalkt und ausgepresst, wodurch das Material zumindest teilweise mechanisch aufgeschlossen wird und außerdem gegebenenfalls noch eingebundene Nährstoffe für die anaerobe, bakterielle Fermentation zugänglich gemacht werden. Die Schneckenpresse kann dabei mobil ausgestaltet sein, beispielsweise auf einem Tieflader angeordnet sein, so dass sie auf dem Fermentervorplatz zu dem betreffenden Fermenter gefahren werden kann.

In einer vorteilhaften Weiterbildung für den thermischen Aufschluss ist ferner eine Einrichtung zur Thermodruckhydrolyse vorgesehen, mit der das Material sowohl vor als auch nach Durchlauf der Feststoffvergärungsanlage aber vor der Gärrestentsorgung einer Thermodruckhydrolyse unterzogen werden kann. Die Thermodruckhydrolyse findet unter ähnlichen Bedingungen statt, wie die eingangs beschriebene Sattdampfbehandlung, jedoch für eine längere Zeitdauer von beispielsweise 60 bis 120 Minuten. Bei der Thermodruckhydrolyse wird das Lignin vollständig ausgelöst, so dass sich eine sirupartige Suspension bildet. Außerdem findet eine echte Hydrolyse, das heißt, eine Aufspaltung von Polymeren in Monomere durch physikalische Einwirkung von Wasser und Hitze statt. Die sirupartige Suspension kann dann in den Fermentationsprozeß (zurück-)gegeben werden oder an Unternehmen verkauft werden, die aus diesem Material Kraftstoffe der zweiten Generation herstellen. Dabei wird auch der Ligninanteil verwertet sowie weitere, unverwertete organische Substanz. Dies ist bei einer anaeroben, bakteriellen Vergärung nicht möglich. Die Figuren zeigen :
- Fig. 1: einen Aufriss eines Biomassekraftwerks nach einer Weiterbildung der Erfindung von Westen betrachtet,
- Fig. 2: einen Aufriss des Biomassekraftwerks von Fig. 1 von Norden betrachten,
- Fig. 3: einen Aufriss des Biomassekraftwerks von Fig. 1 von Süden betrachtet,
- Fig. 4: einen Aufriss des Biomassekraftwerks von Fig. 1 von Osten betrachtet,
- Fig. 5: eine Querschnittsansicht des Biomassekraftwerks von Fig. 1 in westlicher Ansicht,
- Fig. 6: einen Grundriss des Erdgeschosses des Biomassekraftwerks von Fig. 1,
- Fig. 7: einen vergrößerten Ausschnitt aus dem Grundriß von Fig. 6, der eine Strom- und Wärmeerzeugungsanlage zeigt,
- Fig. 8: einen vergrößerten Ausschnitt aus der Grundrissdarstellung von Fig. 6, der einen Anliefer- und Verladebereich zeigt,
- Fig. 9: einen Grundriss des Obergeschosses des Biomassekraftwerks von Fig. 1,
- Fig. 10: eine schematische Darstellung zweier Ansichten eines perforierten Strohballens,
- Fig. 11: eine schematische Querschnittsansicht einer Einrichtung zur Sattdampfbehandlung,
- Fig. 12: eine schematische Querschnittsansicht einer weiteren Einrichtung zur Sattdampfbehandlung, die für loses ligninhaltiges Material bestimmt ist,
- Fig. 13: eine schematische Querschnittsansicht einer Einrichtung zur Sattdampfbehandlung, die zur Sattdampfbehandlung von ballenfönnigem Material bestimmt ist,
- Fig. 14: eine schematische Querschnittsansicht einer Einrichtung zur Sattdampfbehandlung, die eine Mehrzahl von Druckbehältern umfasst.

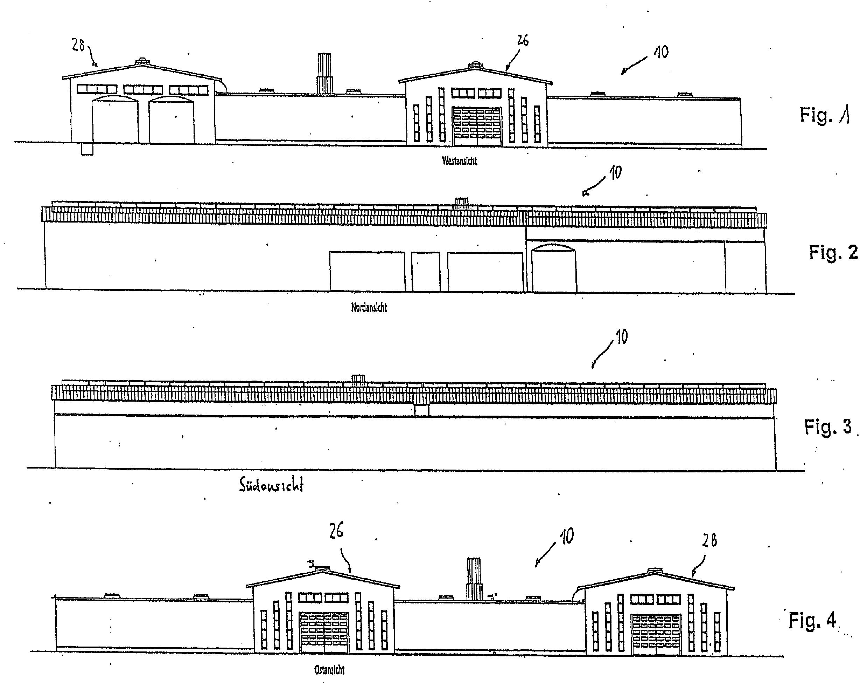

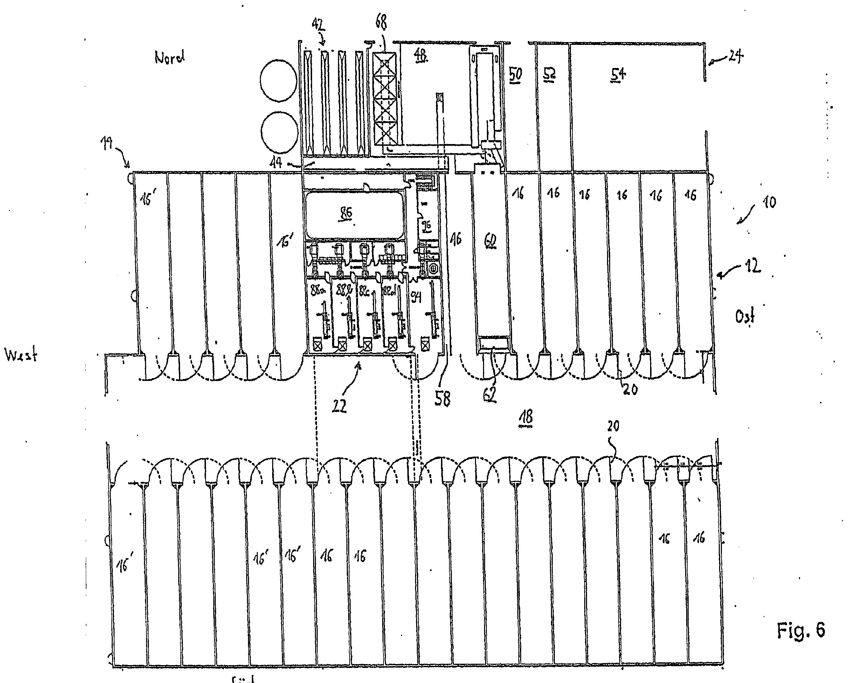

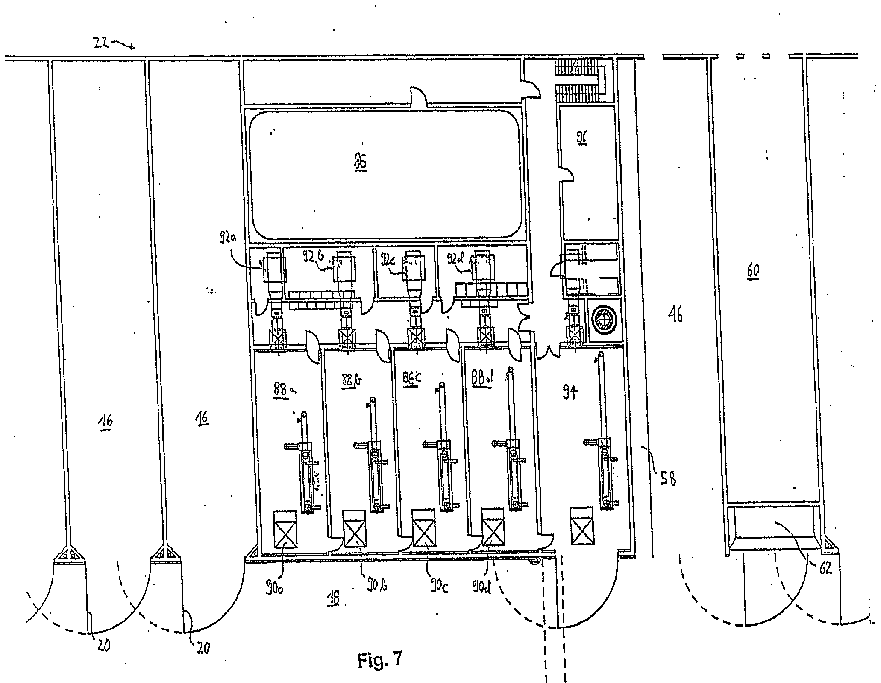

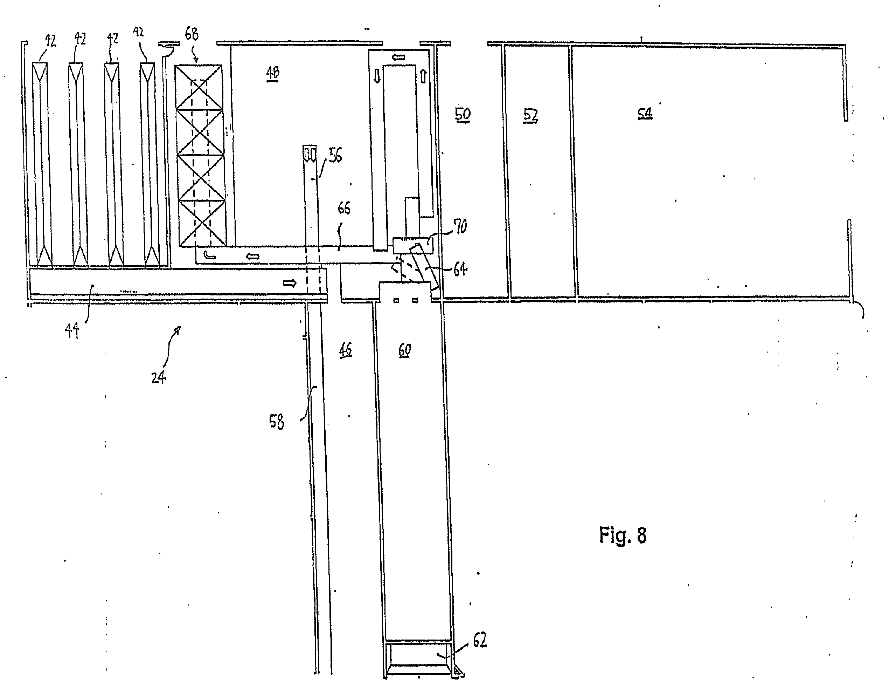

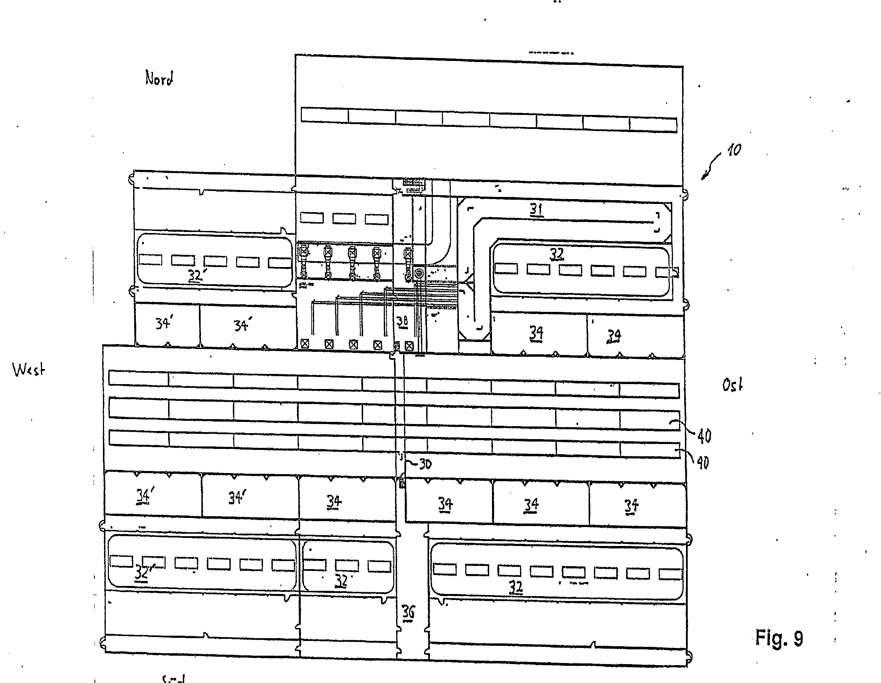

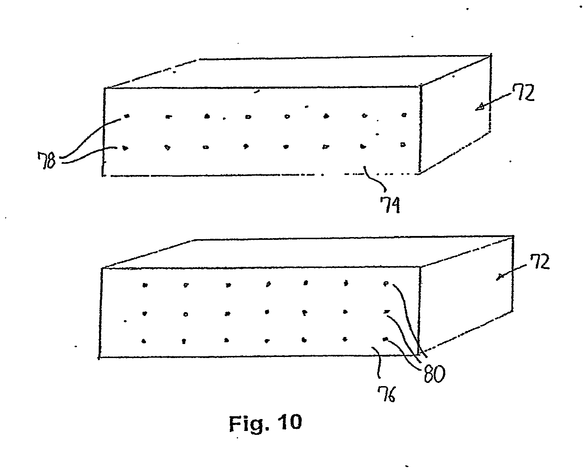

Im Folgenden wird ein Biomassekraftwerk (BMKW) 10 beschrieben. Dabei zeigen die Fig. 1 bis 4 vier Außenansichten des BMKW 10 und Fig. 5 einen Querschnitt desselben. In Fig. 6 ist der Grundriss des Erdgeschosses des BMKW 10 gezeigt. Fig. 7 zeigt einen vergrößerten Teilbereich des Grundrisses von Fig. 10, in dem eine Strom- und Wärmeerzeugungsanlage des BMKW gezeigt ist. Fig. 8 zeigt einen anderen Teilausschnitt des Grundrisses von Fig. 6, in dem ein Anliefer- und Verladebereich vergrößert dargestellt ist. Fig. 9 zeigt eine Grundrissansicht des Obergeschosses des BMKW 10.

Unter Bezugnahme auf den Grundris von Fig. 6 gliedert sich das BMKW 10 in einen Grundabschnitt 12 und einen Ausbauabschnitt 14. Der Grundabschnitt 12 umfasst 18 Fermenter des Garagentyps, die in zwei Reihen angeordnet sind, in der Darstellung von Fig. 5 in einer nördlichen und einer südlichen Reihe. Zwischen den beiden Reihen von Fermentern 16 befindet sich ein Fermentervorplatz 18, zu dem sich die Tore 20 der Fermenter 16 öffnen. Man beachte, dass der Übersicht halber nicht alle Fermenter 16 und Fermentertore 20 in den Figuren mit Bezugszeichen versehen sind.

Ferner umfasst der Grundabschnitt 12 eine Strom- und Wärmeerzeugungsanlage 22, die in Fig. 7 vergrößert dargestellt und unten näher im Detail beschrieben wird. Außerdem umfasst der Grundabschnitt 12 einen Anliefer- und Verladebereich 24, der in Fig. 8 vergrößert dargestellt ist und ebenfalls unten näher beschrieben wird.

Wie den Fig. 1 bis 6 zu entnehmen ist, ist der gesamte Grundabschnitt 12 durch eine Hallenstruktur eingehaust, zu der insbesondere ein Fermentervorplatz (FVP)-Hallenabschnitt 26 und ein Anliefer- und Verladebereichshallenabschnitt 28 gehört, wie insbesondere in Fig. 1, 4 und 5 gut zu erkennen ist. Die gesamte Hallenkonstruktion oder Einhausung des Grundabschnitts 12 wird durch eine große zentrale Entlüftungsanlage entlüftet, so dass im Inneren der Hallenkonstruktion stets ein leichter Unterdruck gegenüber dem Atmosphärendruck herrscht.

Der Ausbauabschnitt 14 besteht im Wesentlichen aus 11 zusätzlichen Fermentern 16' und einer Verlängerung des FVP-Hallenabschnitts 26. Der Ausbauabschnitt 14 dient dazu, bei Bedarf bis zu 11 zusätzliche Fermenter 16' vorzusehen. Das bedeutet, dass das BMKW 10 zunächst ohne den Ausbauabschnitt 14 gebaut und in Betrieb genommen würde. Im Betrieb stellt sich dann heraus, ob die vorhandenen 18 Fermenter 16 des Grundabschnitts 12 ausreichend Biogas erzeugen, um die vier Gasmotoren (nicht gezeigt), die für das BMKW 10 bestimmt sind, unter Volllast mit Gas zu versorgen. Falls dies nicht der Fall ist, kann die entsprechende Anzahl Fermenter 16' im Ausbauabschnitt 14 ergänzt werden, der somit auch kleiner ausfallen kann als in Fig. 6 gezeigt. Mit anderen Worten hat das BMKW 10 einen modularen Aufbau, der zum Erreichen einer optimalen Endkonfiguration vorteilhaft ist, weil der exakte Biogasertrag von einer Vielzahl von Faktoren, darunter die Beschaffenheit der vorhandenen Frischmasse, abhängt und nicht theoretisch exakt vorhersagbar ist.

Die nördliche und die südliche Fermenterreihe sind durch eine Technikbrücke 30 verbunden, die insbesondere in Fig. 5, 6 und 9 zu sehen ist. Die Technikbrücke 30 überspannt den FVP 18 in einer Höhe, die gestattet, dass Radlader, von denen in Fig. 5 zwei exemplarisch gezeigt sind, unter ihr selbst mit voll ausgefahrener Ladeschaufel hindurch fahren können, ohne dass sie die Technikbrücke berühren und beschädigen können.

Unter Bezugnahme auf Fig. 9 umfasst das Obergeschoß des BMKW 10 drei Folien-Gasspeicher 32 im Grundabschnitt 12 und zwei weitere Foliengasspeicher 32' im Ausbauabschnitt 14. Die Foliengasspeicher 32 sind in den Querschnittsansichten von Fig. 5 und 15 gut zu erkennen. Sie nehmen auf nachfolgend näher beschriebene Weise das Biogas auf, welches in den Fermentern 16 bzw. 16' erzeugt wird.

Ferner umfasst das Obergeschoß fünf Perkolatumlauftanks (PUT) 34 im Grundabschnitt 12 und vier PUTs 34' im Ausbauabschnitt 14, die ebenfalls in den Querschnittsansichten von Fig. 5 gut zu sehen sind. Ein PUT 34 ist jeweils oberhalb von drei Fennentern 16 angeordnet und empfängt von ihnen Perkolat, welches am Boden der Fermenter gesammelt und in den PUT 34 gepumpt wird. Mit dem Begriff "Perkolat" bezeichnet man den flüssigen, gülleähnlichen Bestandteil der Gärsubstanz.

Ferner befindet sich im Obergeschoß ein Abgas-Abkühlraum 31, ein südlicher Technikraum 36 und ein nördlicher Technikraum 38, die durch die Technikbrücke 30 miteinander verbunden sind. Im FVP-Hallenabschnitt 26 und im Anliefer- und Verladebereichshallenabschnitt 28 sind ferner Lichtbänder 40 angeordnet.

Nachdem ein grober Überblick über die Bestandteile des BMKW 10 gegeben wurde, werden im Folgenden die einzelnen Abschnitte und Komponenten sowie deren Betriebsweise im Detail beschrieben.

### 1. Fermentervorplatz

Der Fermentervorplatz (FVP) 18 ist im Zentrum den BMKW 10 angeordnet. Er dient als Transportweg für Frischmasse zu den jeweiligen Fermentern 16, 16' bzw. von Gärrestesubstanz aus den Fermentern 16, 16'. Außerdem dient er als Anmischfläche, auf der der Inhalt eines Fermenters ausgebreitet wird, von dem etwa ein Fünftel bis ein Viertel als Gärrest entnommen wird und zum Ausgleich dieser Entnahme und des durch die Vergasung entstehenden Masseverlusts etwa ein Drittel durch Frischmasse ergänzt und mit der alten Gännasse vermischt wird. Diese Arbeit kann auf dem FVP 18 von einem großen Radlader verrichtet werden, wie er in Fig. 5 schematisch dargestellt ist. In der Mitte des FVPs 18 befindet sich eine mit einem Gitterrost versehene Ablaufrinne, in die Sickersäfte und freigesetztes Perkolat fließen. Auf der Höhe der Technikbrücke 30 hat die Ablaufrinne einen Sammelschacht (nicht gezeigt) aus dem die anfallenden Flüssigkeiten über eine Perkolat-Ringleitung (nicht gezeigt) zu einem der PUTs 34 gefördert werden.

### 2. Anliefer- und Verladungsbereich

Der Anliefer- und Verladungsbereich 24 ist in Fig. 8 im Grundriss vergrößert dargestellt. Im gezeigten Ausführungsbeispiel wird, was die Anlieferung betrifft, zwischen loser Frischmasse und Strukturfrischmasse bzw. Ballenfrischmasse unterschieden. Für die lose Frischmasse sind in der gezeigten Ausführungsform vier Anlieferbunker 42 vorgesehen, die durch den Anliefer- und Verladebereichshallenabschnitt 28 eingehaust sind. Ein LKW kann rückwärts in den eingehausten Anlieferbunker rangieren und die Frischmasseladung dort in die Anlieferbunker 42 abkippen oder abschieben. Da im gesamten Anliefer- und Verladebereich 24 ein leichter Unterdruck herrscht, treten kaum störende Gerüche aus der Einhausung nach außen aus. Jeder Anlieferbunker 42 hat einen nach unten konisch zulaufenden Boden, an dessen tiefster Stelle eine oder mehrere Doppelförderschnecke (nicht gezeigt) vorgesehen sind, die die Frischmasse horizontal bis zu einem Becher-Elevator (nicht gezeigt) fördert, welcher die Frischmasse auf ein Förderband 44 befördert, oder direkt auf ein tiefergelegenes Förderband.

Von dem Förderband 44 wird die Frischmasse in einen Frischmassebunker 46 fallengelassen. Da die Frischmasse aus vier oder mehr verschiedenen Bunkern auf demselben Förderband 44 befördert wird und auf denselben Haufen in dem Frischmassebunker 46 geworfen wird, wird die Frischmasse automatisch durchmischt.

Der Frischmassebunker 46 ist eine längliche Kammer, die den Anliefer- und Verladebereich 24 mit dem Fermentervorplatz 18 verbindet, wie insbesondere in Fig. 6 zu sehen ist. Der Frischmassebunker 46 hat eine Fußbodenheizung, mit der die Frischmasse bereits auf eine Temperatur von 42°C erwärmt wird, damit die Gärmasse innerhalb eines Fermenters 16, 16', welche durch die Frischmasse ergänzt wird, durch diese nicht abgekühlt wird, so dass der Gärprozess nach Verschließen des Fermenters 16 wieder schnell startet und ggf. auch bereits eine schwach aerobe Vorhydrolyse stattfinden kann, die die Gärzeit verkürzt und die Anlagenleistung (Durchsatz an Gärsubstrat) sowie die Wirtschaftlichkeit der Anlage erhöht.

Der Frischmassebunker 46 hat eine doppelte Funktion. Zum einen dient er als Zwischen- oder Pufferspeicher für lose Frischmasse. Zum anderen dient er als Transportweg zwischen dem Anliefer- und Verladebereich 24, also der Peripherie des BMKWs 10, und dem zentral gelegenen Fermentervorplatz 18. Zum Transport ist in dem Frischmassebunker 46 ein Schubschild bzw. Schieber angeordnet (nicht gezeigt) der jeweils neu von oben eingeschüttete lose Frischmasse in Richtung auf den Fermentervorplatz 18 schiebt. Dann wird der Schieber zurückgefahren, um Platz für neue Frischmasse zu machen. Durch diesen Schiebemechanismus wird erreicht, dass die Frischmasse ungefähr in der gleichen Reihenfolge, in der sie am Eingang in den Frischmassebunker 46 eingeworfen wurde, auch auf der Seite des Fermentervorplatzes 18 aus ihm herausgeschoben wird. Dies bedeutet, dass die Frischmasse, die zum Fermentervorplatz 18 gelangt, immer ungefähr gleich alt und daher von konstanter Beschaffenheit ist, was für die anschließende Vergärung vorteilhaft ist.

Ferner umfasst der Anliefer- und Verladebereich 24 einen Abschnitt für das Anliefern und Transportieren von Struktur- bzw. Ballenmaterial von Stroh. Dieser Abschnitt zum Anliefern und Transportieren von Ballenmaterial umfasst einen Bereitstellungsraum 48, einen Ballen-Anlieferungsraum 50, einen Aufschlussbereich 52 und ein Zwischenlager 54. Im Folgenden wird dieser Bereich des Anliefer- und Verladebereichs 24 unter Bezugnahme auf Stroh als stark lignifiziertes, ballenförmiges Strukturmaterial beschrieben, aber es versteht sich, dass dieser Abschnitt auch zur Anlieferung, Bearbeitung und den Weitertransport von anderem ballenfönnigen Strukturmaterial verwendet werden kann.

Ein Kran (nicht gezeigt) ist an einer Laufschiene so angebracht, dass er Strohballen in jedem der Räume 48 bis 54 aufgreifen und ablegen kann. Die Strohballen werden im Strohanlieferungsraum 50 angeliefert und von dem Kran (nicht gezeigt) in das Zwischenlager 54 transportiert. Bevor das Stroh zum Fennentervorplatz 18 befördert wird, wird es in dem Aufschlussbereich 52 vorbehandelt, nämlich aufgeschlossen. Der Aufschluss des Strohs ist nötig, da das Stroh stark lignifiziert ist, und die Bakterien im Fermenter 16 aufgrund der ligninkrustierten Cellulose nur sehr schlecht an die vom Lignin eingeschlossenen Nährstoffe gelangen. In dem Aufschlussbereich 52 kann das Stroh je nach Ausführungsform des BMKW 10 auf unterschiedliche Arten aufgeschlossen werden. Beispielsweise kann das Stroh chemisch aufgeschlossen werden, indem es in einem Behälter eingeweicht wird, welcher Wasser, eine Wasser-Laugen-Lösung oder eine Wasser-Säure-Lösung enthält. Durch die Einweichung wird das Lignin, das die Cellulose weitgehend eingeschlossen hat, teilweise aufgelöst. Nach der Entnahme aus dem Behälter ist die Cellulose nicht mehr hinter einer Ligninkruste verborgen, sondern für die Hydrolyse und die Bakterien zugänglich. Dadurch wird das Stroh, welches in herkömmlichen Nass- oder Trockenvergärungsanlagen bisher nur als Strukturmaterial verwendet wird, zu einem wertvollen Gärsubstrat, welches zur Biogasentwicklung wesentlich beiträgt.

In einer alternativen Ausführungsform kann das Stroh in dem Aufschlussbereich 52 aber auch auf andere Weise aufgeschlossen werden, beispielsweise mechanisch unter Verwendung einer Hammermühle oder indem es einem Thermodruck ausgesetzt wird, d.h. bei hohem Druck von beispielsweise 20 bis 30 bar für fünf bis zehn Minuten auf 180°C bis 250°C erhitzt wird. Dabei weicht das Lignin auf. Nach dem Erkalten des Strohs erstarrt das Lignin zwar wieder, jedoch in Form sehr kleiner Kügelchen mit Zwischenräumen dazwischen, die den Weg für die autohydrolytischen, organischen Säuren und für die anaeroben Bakterien zu den Nährstoffen des Strohs freigeben. Ein weiteres Ausführungsbeispiel ist eine Erweiterung der Thermodruckbehandlung, bei der der Druck in dem entsprechenden Behälter plötzlich reduziert wird, wodurch das Wasser in den Strohstrukturen verdampft und sich sehr rasch ausdehnt. Dabei werden die Ligninstrukturen zerrissen und die Nährstoffe für die anaeroben Bakterien freigelegt. Die weiteren Details des Strohaufschlusses werden im nachfolgenden Abschnitt angegeben.

Im Bereitstellungsraum 48 ist ein Rollenförderer 56 vorgesehen, auf den einzelne Strohballen und/oder Strohballenpakete durch den Kran (nicht gezeigt) aufgelegt werden, und der die Strohballen durch einen Strohkanal 58, der parallel zum Frischmassebunker 46 angeordnet ist, zum Fermentervorplatz 18 befördert (siehe Fig. 6).

Wie der obigen Beschreibung zu entnehmen ist, wird sowohl die lose Frischmasse als auch die ballenförmige Frischmasse durch stationäre Fördertechnik von dem Anliefer- und Verladebereich 24 zum Fermentervorplatz 18 befördert. Insbesondere stellen dabei der Frischmassebunker 46 und der Strohkanal 58 die Verbindung zwischen dem zentralen Fermentervorplatz 18 und dem peripheren Anliefer- und Verladebereich 24 bereit, und dieser Transport geschieht vollständig im eingehausten BMKW 10. Der Transport mit der stationären Fördertechnik ist für hohe Durchsätze geeignet und insbesondere schneller, platzsparender und kostengünstiger als eine Anlieferung mit Radladern. Wie Fig. 6 zu entnehmen ist, enden der Strohkanal 58 und der Frischmassebunker 46 an einer zentralen Stelle des Fermentervorplatzes 18, so dass die Wege zwischen dem fermentervorplatzseitigen Ende des Frischmassebunkers 46 bzw. Strohkanals 58 und dem zu beliefernden Fermenter 16 im Allgemeinen kurz sind.

Wie oben erwähnt wurde, ermöglicht der Aufschluss des Strohs im Aufschlussbereich 52 es, Stroh trotz seines hohen Ligningehaltes als Gärsubstrat zu verwenden. Dies ist äußerst vorteilhaft, weil Stroh bei der Getreideproduktion ohnehin anfällt, und gar nicht ausreichend Verwendung für dieses besteht. Da das BMKW 10 für nachwachsende Rohstoffe konzipiert ist, bietet es sich an, in der Umgebung des BMKW 10 speziell zur Verwendung im BMKW 10 geeignete Rohstoffe anzubauen, die jedoch im Allgemeinen nicht für die Nahrung bestimmt sind. Dies stellt jedoch einen gewissen Ziel-Konflikt dar, weil stets ein bestimmter Anteil von den begrenzten zur Verfügung stehenden Flächen für die Nahrungsproduktion reserviert ist. Die Verwendung von Stroh als Gärsubstrat stellt eine sehr attraktive Lösung dar, da Stroh, welches bei der Getreideproduktion anfällt, die gleichzeitige Produktion von Nahrungsmitteln und kraftwerkstauglicher Biomasse erlaubt.

Stroh hat aber noch einen weiteren Vorteil. Im Allgemeinen ist die Füllhöhe in Fermentern durch den Druck begrenzt, der sich am Fermenterboden ergibt: Dieser Druck muss stets so niedrig sein, dass das Gärsubstrat noch durchlässig für Perkolat ist. Wenn man hingegen, gemäß einer Weiterbildung der Erfindung, in der untersten Lage eines jeden Fermenters 16 eine Schicht aus Strohballen einbringt, kann auf diese Schicht noch die gesamte übliche Füllhöhe an Gärsubstanz geschichtet werden, da die Strohballenschicht auch bei dem dann auftretenden Druck noch durchlässig für Perkolat ist. Diese unterste Strohschicht stellt also eine zusätzliche Menge an Gärsubstrat dar, die in einem Fermenter verwendet werden kann, so dass die Anlagenleistung (Raumleistung gemessen in neuem Substrat pro Fermenter und Tag) erheblich gesteigert wird.

In einer vorteilhaften Weiterbildung der Erfindung werden die Strohballen in Paketen aus acht Strohballen auf den Rollenförderer 56 aufgelegt, die zwei Ballen breit und vier Ballen hoch sind. Diese Pakete werden als Ganzes durch den Strohkanal 58 transportiert und an dessen Ende am Fermentervorplatz 18 von einem Umsetzer (nicht gezeigt) abgehoben und an einen Radlader oder Gabelstapler übergeben, der die Pakete ebenfalls als Ganzes oder in 2 Teilen empfängt und zum Fermenter bringt. Aus diesen Paketen kann relativ einfach und zügig die besagte unterste Strohballenschicht aufgebaut werden.

Wie weiter in Fig. 8 zu sehen ist, ist ein Gärrestebunker 60 vorgesehen, der sich parallel zum Frischmassebunker zwischen dem Fermentervorplatz 18 und dem Anliefer- und Verladebereich 24 erstreckt. Der Gärrestebunker 60 hat an seinem dem Fermentervorplatz zugewandten Ende ein Schütttrog 62 für Gärreste, die den Eingang in den Gärrestebunker 60 bildet. In diesen Schütttrog 62 werden Gärreste von einem Radlader gekippt. Von dort werden sie mittels einer Förderschnecke in den Gärrestebunker gedrückt. Durch das diskontinuierliche Nachdrücken immer neuer Gärreste wird die Masse langsam durch den Gärrestebunker 60 bis zum anderen Ende befördert, wo sie durch weitere Förderschnecken aus dem Gärrestebunker 60 transportiert werden.

Der Gärrestebunker 60 hat eine dreifache Funktion. Zum einen dient er als Transportweg zwischen dem Fermentervorplatz 18 im Zentrum des BMKW 10 und dem Anliefer- und Verladebereich, mit ähnlichen Vorteilen, wie sie im Hinblick auf den Frischmassebunker 46 und den Strohkanal 58 beschrieben wurden. Zum anderen dient der Gärrestebunker 60 aber auch als thermophil betriebener Nachgärer und wirkt quasi wie ein weiterer Fermenter. Daher ist der Gärrestebunker 60 auch an das Biogassystem angeschlossen.

Schließlich dient der Gärrestebunker 60 als Zwischenspeicher für Gärreste. Er ist so dimensioniert, dass er mindestens so viel Gärreste fasst, wie an zwei Tagen anfallen können. Dies ermöglicht es, den Abtransport der anfallenden Gärreste an Wochentagen vorzunehmen, so dass der Abtransport nicht durch LKW-Fahrverbote behindert ist.

Am Austrittsende des Gärrestebunkers 60 ist eine Weiche 64 vorgesehen, die es ermöglicht, die Gärreste entweder direkt über ein Förderband 66 zu Verladesilos 68 zu transportieren, oder den Umweg über eine Dehydriereinrichtung 70 zu nehmen. Im gezeigten Beispiel ist die Dehydriereinrichtung 70 eine Schneckenpresse, die geeignet ist, Wasser bzw. Perkolat aus den Gärresten zu pressen und in einen der PUTs 34 einzuführen. Ob der Umweg über die Dehydriereinrichtung 70 genommen wird, hängt von dem aktuellen Bedarf an Perkolat ab.

Die Verladesilos für Gärreste 68 sind Hochsilos, die auf Trapezgestellen angeordnet sind, so dass ein LKW unter die Silos 68 fahren kann und somit auf einfache Weise beladen werden kann.

In einer alternativen Ausführungsform ist eine konventionelle Trocknungsanlage, beispielsweise ein Trommel- oder Bandtrockner (nicht gezeigt) vorgesehen, die geeignet ist, die Gärreste auf unter 25 % vorzugsweise auf 15% Wasseranteil zu trocknen. Die Wärme für die Trocknungsanlage wird dabei vorzugsweise durch die Abwärme von den Generatorsets bereitgestellt. Ferner ist eine Vergasungsanlage (nicht gezeigt) vorgesehen, in der die getrockneten Gärreste einer sogenannten Holzvergasung unterzogen werden, bei der aus den getrockneten Gärresten durch Pyrolyse oder Teilverbrennung unter Luftmangel brennbares Holzgas (Schwachgas) gewonnen wird. Dieses Holz- bzw. Schwachgas wird nach der Entfernung des anfallenden Teers aus dem Holzgas nach irgendeinem bekannten Verfahren in das Biogassystem eingespeist und kann dann völlig unproblematisch als Treibstoff für die Gas-Ottomotoren eingesetzt werden.

Der Energiegehalt des Holzgases reduziert den Bedarf an Biogas um bis zu 20 %, ggf. auch mehr, so dass für dieselbe Stromleistung bis zu 20 % ggf. auch bis zu 30% weniger Substrat für die Vergärung eingesetzt werden muss. Dadurch steigt die Wirtschaftlichkeit der Anlage als Ganzes erheblich.

### 3. Strohaufschluss

Wie eingangs erwähnt, wird bei dem gezeigten Biomasse-Kraftwerk das Stroh im Anliefer- und Verladebereich 24 angeliefert und im Aufschlussbereich 52 und eventuell zusätzlich im Bereitstellungsraum 48 aufgeschlossen. In der Erfindung wird Stroh als LHNawaRo in Ballenform angeliefert und auch in Ballenform aufgeschlossen, um schließlich in Ballenform in die Garagenfermenter 16 eingebracht zu werden. Die Ballen haben dabei vorzugsweise eine Dichte von über 200 kg/m³, die sich nur mit Höchstdruckballenpressen erzeugen lassen. Der Vorteil einer solch hohen Dichte der Strohballen besteht darin, dass dann die Kapazität eines LKW sowohl bezüglich des zulässigen Gewichtes der Ladung als auch des möglichen Volumens der Ladung optimal ausgeschöpft wird, so dass das Stroh auch über weitere Strecken zu wirtschaftlichen Bedingungen geliefert werden kann.

In der hier gezeigten Ausführungsform umfasst die Vorbehandlung zum Aufschluss des Strohs vier Schritte, die in dem Aufschlussbereich 52 bzw. dem Bereitstellungsraum 48 durchgeführt werden, nämlich
1.) Perforieren bzw. Lochen der Strohballen,
2.) Einweichen der Strohballen in Wasser,
3.) Sattdampfbehandlung der eingeweichten Strohballen, und
4.) Einweichen der Strohballen in Perkolat.

Diese Schritte und die dabei eingesetzten Vorrichtungen werden im Folgenden beschrieben.

Parallel kann ein Teil des Strohs in die hier beschriebene Aufschlussform "Vermahlung" und/oder in die erfindungsgemäße Aufschlussform "Thermodruckhydrolyse" gegeben werden. Die Kombination der verschiedenen Aufschlussformen ist besonders vorteilhaft, denn jede hat ihre Vor- und Nachteile für den Praxisbetrieb. Durch die Kombination wird der beste Gesamteffekt erzielt.

Wie beschrieben, ist es möglich, unabhängig von der Vorbehandlung des übrigen Strohs (oder LHNawaRo im Allgemeinen), einen Teil des Strohs zu vermahlen, insbesondere zu einer Pulverkonsistenz, und der übrigen Frischmasse beizumischen. Das vermahlene Stroh führt zu einem besonders hohen Gasertrag, jedoch ist der Anteil im Verhältnis zur Frischmasse insofern begrenzt, als das durch Perkolat benetzte pulverisierte Stroh eine klebrige Masse bildet, die wegen der Handhabbarkeit mit ausreichend Frischmasse gemischt werden muss.

Wie beschrieben, besteht zwischen 5 und 25 Gewichtsprozent des Gärsubstrates als Ganzes aus gemahlenem Stroh. Wie beschrieben, werden zwischen 5% und 35% des insgesamt verwendeten Strohs vermahlen und dadurch mechanisch aufgeschlossen.

Ferner ist es vorteilhaft, unabhängig von der Vorbehandlung des übrigen Strohs (oder LHNawaRo im Allgemeinen) 5-20% des insgesamt verwendeten Strohs in einer Thermodruckhydrolyse aufzuschließen und das dabei erhaltene, sirupähnliche Material, das sogenannte "slurry" in den Perkolatkreislauf einzubringen.

Schließlich ist es vorteilhaft, unabhängig von den übrigen Verfahrensschritten zum Strohaufschluss das aus dem Fermenter entnommene Gärsubstrat mechanisch durchzupressen.

### 3.1. Perforation

Die Perforation der Strohballen dient dazu, das Innere des Strohballens für das Einweichen, für die Sattdampfbehandlung und für die nachfolgende Einweichung in Perkolat zugänglich zu machen. In der hier beschriebenen Ausführungsform werden die Strohballen von zwei Seiten gelocht, wie unter Bezugnahme auf Fig. 10 näher erläutert wird.

Fig. 10 zeigt oben eine perspektivische Ansicht eines Strohballens 72, in der auf dessen Unterseite 74 geblickt wird. Die untere Abbildung zeigt eine perspektivische Ansicht desselben Strohballens 72, bei der auf dessen Oberseite 76 geblickt wird. Von der Unterseite 74 aus ist der Strohballen 72 durch einen ersten Satz Löcher 78 perforiert, die nicht durch den gesamten Ballen 72 hindurchgehen. Ferner ist der Strohballen 72 von der Oberseite 76 aus mit einem zweiten Satz Löcher 80 perforiert, die ebenfalls nicht durch den gesamten Strohballen 72 hindurchgehen. Die Löcher 78 und 80 sind so gegeneinander versetzt, dass die Löcher des ersten Satzes 78 und die Löcher des zweiten Satzes 80 durch Materialbrücken voneinander getrennt sind. Durch diese Art der Lochung kann das Einweichwasser des zweiten Schritts, der Sattdampf des dritten Schritts und das Perkolat des vierten Schritts gut in das Innere des Strohballens 72 eindringen, ohne auf der anderen Seite wieder herauszulaufen.

### 3.2. Einweichen

In dem Aufschlussraum 52 von Fig. 8 sind geeignete Behälter zum Einweichen von Strohballen vorgesehen, die in der Darstellung nicht gezeigt sind. Die Größe der Behälter zum Einweichen ist auf die Ausmaße der Strohballen abgestimmt, so dass das Einweichen platzsparend und effizient durchgeführt werden kann.

### 3.3. Sattdampfbehandlung

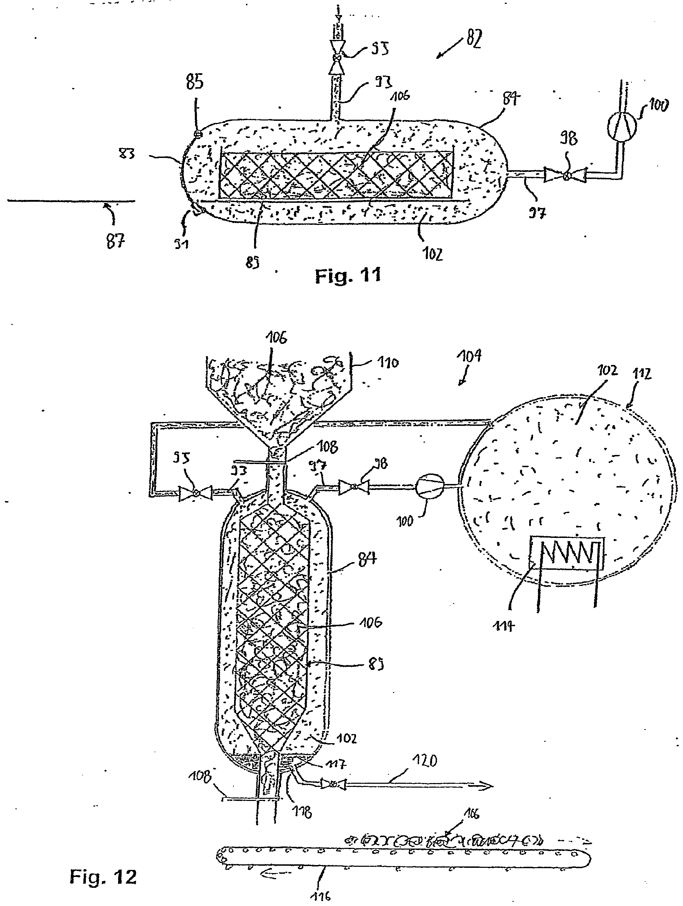

Im Aufschlussbereich 52 oder im Bereitstellungsraum 48 ist eine Einrichtung zur Sattdampfbehandlung vorgesehen. Unter Bezugnahme auf Fig. 11 bis 14 werden verschiedene Einrichtungen zur Sattdampfbehandlung beschrieben, die in der gezeigten Anlage oder einer abgewandelten Anlage verwendet werden können.

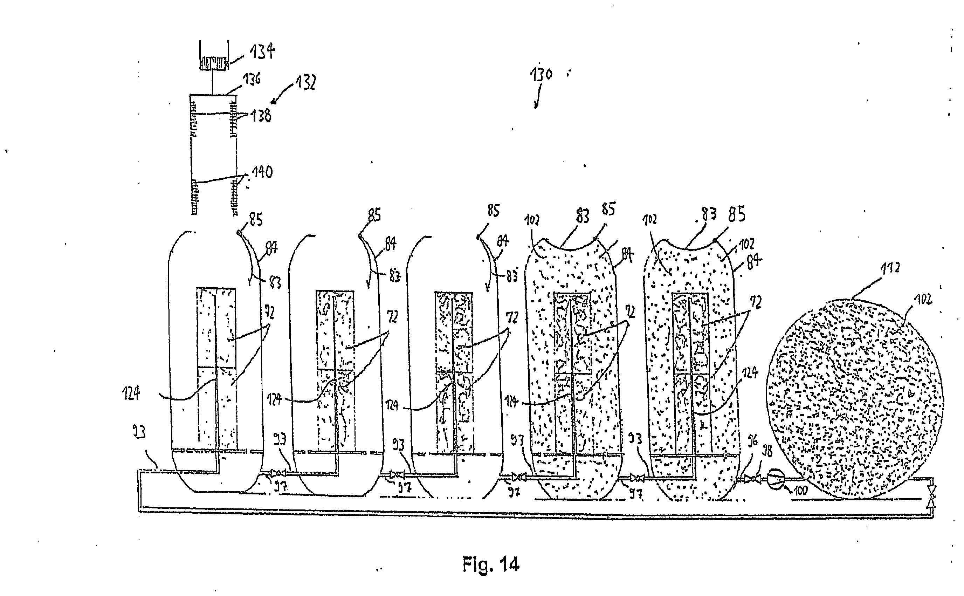

Fig. 11 zeigt in einer schematischen Querschnittsansicht einen einfachen Aufbau einer Einrichtung 82 zur Sattdampfbehandlung. Die Einrichtung 82 umfasst einen Druckbehälter 84 mit einem Deckel 83, der über ein Gelenk 85 schwenkbar am Druckbehälter 84 angelenkt ist. Eine Zuführeinrichtung für das Stroh ist schematisch angedeutet und durch Bezugszeichen 87 bezeichnet. Da mit der Einrichtung 82 zur Sattdampfbehandlung Strohballen behandelt werden sollen, kann die Zuführeinrichtung 87 beispielsweise durch ein Förderband oder einen Rollenförderer gebildet werden. Falls die Einrichtung 82 für lose LHNawaRos, beispielsweise für hierin beschriebenes loses Stroh 106, verwendet werden soll, kann die Zuführeinrichtung 87 aus Schienen bestehen, auf denen ein Behälter 89 für loses Material in den Druckbehälter 84 geschoben werden kann. Der Behälter 89 ist für Wasserdampf durchlässig, aber geeignet, das lose Material zu halten und kann beispielsweise ein oben offener Gitterbehälter bzw. Korb sein. Der Deckel 83 des Druckbehälters 84 kann durch einen Schließmechanismus 91 verschlossen werden. Vorzugweise wird der Deckel 83 zum Öffnen des Druckbehälters 84 nach innen geschwenkt, wie dies beispielsweise in Fig. 14 zu sehen ist, so dass der Deckel 83 durch den Druck im Inneren des Druckbehälters 84 in die geschlossene Stellung gedrückt wird und dadurch einfacher abgedichtet wird.

Der Druckbehälter 84 ist mit einer Zufuhrleitung 93 und einem Zuführungs-Ventil 95 verbunden, durch die Sattdampf 102 mit einem Druck von bis zu 30 bar und einer Temperatur von bis zu 250°C von einem Dampfreservoir (nicht gezeigt) in den Druckbehälter 84 eingeführt werden kann. Ferner ist der Druckbehälter 84 mit einer Auslassleitung 97 mit einem Auslass-Ventil 98 verbunden, über die der Dampf nach der Sattdampfbehandlung aus dem Druckbehälter 84 ausgelassen werden kann. Ferner ist in der Auslassleitung 97 ein Kompressor 100 angeordnet, mit dem der Sattdampf 102 in das Reservoir (nicht gezeigt) zurückbefördert werden kann.

Im Folgenden wird das Verfahren der Sattdampfbehandlung unter Bezugnahme auf die Einrichtung 82 zur Sattdampfbehandlung von Fig. 11 erläutert. Zunächst wird das ligninhaltige Material 106 als Ballen in einem Behälter wie in dem Behälter 89 in den Druckbehälter 84 eingebracht und der Druckbehälter 84 geschlossen. Danach wird das Ventil 95 in der Zufuhrleitung 93 geöffnet, so dass heißer Wasserdampf mit einer Temperatur von 180°C bis 250°C und einem hohen Druck von zwischen 20 und 30 bar von einem Dampfreservoir (nicht gezeigt) in den Druckbehälter 84 eingeführt wird. Der eingeführte Sattdampf ist in Fig. 11 schematisch angedeutet und mit dem Bezugszeichen 102 bezeichnet.

Das ligninhaltige Material 106 wird dem Sattdampf 102 für 5 bis 15 Minuten ausgesetzt. Dabei wird das Lignin in dem Material geschmolzen, aber nicht aus dem Material ausgelöst. Für die Effizienz der Sattdampfbehandlung ist es vorteilhaft, wenn das Material, d. h. die Strohballen, in dem oben genannten zweiten Schritt zuvor eingeweicht wurde, weil das Wasser dann bereits im Material vorhanden ist und nur noch erhitzt werden muss, was die Behandlungsdauer verkürzt.

Nach der vorbestimmten Verweildauer von 5 bis 15 Minuten wird der Sattdampf durch die Auslassleitung 97 aus dem Druckbehälter 84 entlassen. Dieses Entlassen des Drucks findet vorzugsweise schlagartig statt, so dasss der Druck innerhalb von 5 Sekunden oder weniger um mindestens 80 % abnimmt. Durch die rasche Absenkung des Drucks verdampft das Wasser in den Strukturen des ligninhaltigen Materials schlagartig und dehnt sich dabei rasch aus. Dabei werden die Ligninstrukturen des Strohs zerrissen, so dass die Nährstoffe (Cellulose und Arabinoxylan) für wässrige organische Säuren und die anaeroben Bakterien zugänglich werden.

Nach dem Entlassen des Drucks aus dem Druckbehälter 84 wird das ligninhaltige Material 106 aus dem Druckbehälter 84 entfernt, und es kühlt sich ab. Beim Abkühlen erhärtet das geschmolzene Lignin wieder. Jedoch bilden sich beim Erstarren des Lignins nicht wieder die ursprünglichen flächigen Strukturen, sondern es koaguliert vielmehr in einer Tröpfchenstruktur, die Zwischenräume lässt, durch die zunächst organische Säuren und dann Bakterien an die Cellulose und das Arabinoxylan (Hemicellulose) gelangen können.

Der in Fig. 11 grundsätzlich gezeigte Aufbau der Einrichtung 82 zur Sattdampfbehandlung kann auf vielfältige Weise modifiziert werden, und einige Beispiele solcher Modifikationen werden im Folgenden angegeben. Dabei werden identische oder funktionell gleiche Komponenten mit denselben Bezugszeichen wie in Fig. 11 bezeichnet, und deren Beschreibung nicht wiederholt.

Die zu Vergleichszwecken aufgeführte Fig. 12 zeigt einen Aufbau einer Einrichtung zur Sattdampfbehandlung, die für eine quasi kontinuierliche Verarbeitung von losem Material ausgebildet ist. Auch hier ist im Inneren des Druckbehälters 84 ein Behälter 89 für loses Material 106 vorgesehen, jedoch ist dieser in dem Druckbehälter 84 fest installiert. Zum Befüllen des Behälters 89 wird ein druckfester Schieber 108 geöffnet, so dass das ligninhaltige Material 106 aus einem Trichter 110 in den Behälter 88 fällt. Wenn eine ausreichende Menge Material 106 im Behälter 89 ist, wird der druckfeste Schieber 108 geschlossen, und die Sattdampfbehandlung findet auf dieselbe Weise statt, wie sie unter Bezugnahme auf Fig. 11 beschrieben wurde. Zusätzlich zu den Komponenten von Fig. 11 ist jedoch in Fig. 12 ein Reservoir 112 für Sattdampf 102 gezeigt, welches über eine Heizung 114 verfügt. Nach der Sattdampfbehandlung wird der Dampf über die Auslassleitung 97 entlassen und über den Kompressor 100 in das Reservoir 112 gedrückt. Danach wird ein weiterer druckfester Schieber 108 am unteren Ende des Behälters geöffnet, und das aufgeschlossene lose Material 106 fällt auf ein Förderband 116 zum Weitertransport.

Am unteren Ende des Behälters 84 sammelt sich insbesondere bei der Thermodruckhydrolyse eine flüssige Masse 117, die man als "Slurry" bezeichnet und die über eine weitere Leitung 118 abgelassen und über eine Leitung 120 in die Perkolatumlauftanks (nicht gezeigt) geleitet wird.

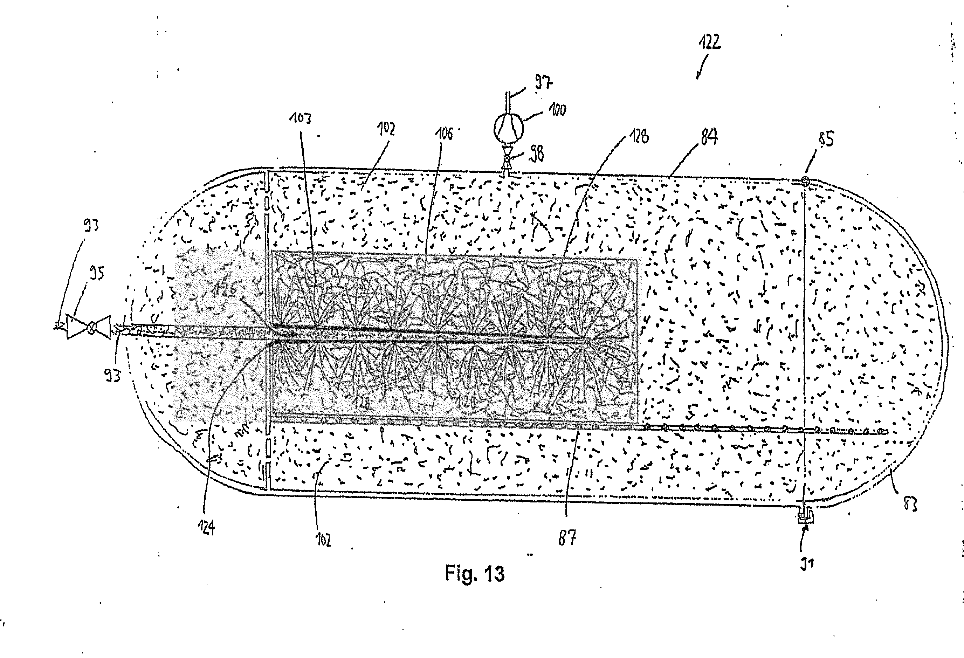

Fig. 13 zeigt eine erfindungsgemäße Ausführungsform 122 einer Einrichtung zur Sattdampfbehandlung, die speziell für die Behandlung von Ballenmaterial, d. h. Strohballen 72, ausgelegt ist. Der Aufbau ist grundsätzlich ähnlich zum Aufbau von Fig. 11 und wird nicht erneut beschrieben. Der wesentliche Unterschied besteht jedoch darin, dass ein Spieß oder Dorn 124 vorgesehen ist, der einen inneren Hohlraum 126 und mit diesem inneren Hohlraum 126 verbundene düsenartige Öffnungen 128 hat. Der innere Hohlraum 126 ist mit der Zufuhrleitung 93 in Fluidverbindung.

Im Betrieb der Einrichtung zur Sattdampfbehandlung 122 von Fig. 13 wird ein Strohballen 72 bzw. 106 über die Zuführeinrichtung 87, die in der gezeigten Ausführungsform durch eine Rollenbahn gebildet wird, in der Darstellung von Fig. 13 von rechts in den Druckbehälter 84 eingebracht und auf den Spieß 124 aufgespießt. Dann wird der Druckbehälter 84 wie gehabt verschlossen, und der Sattdampf 102 wird über die Zufuhrleitung 93, den inneren Hohlraum 126 des Spießes 124 und die düsenartigen Öffnungen 128 in den Strohballen 72 injiziert. Dadurch wird erreicht, dass auch das Innere des Strohballens 72 wirksam mit dem Sattdampf in Kontakt kommt. Wird nämlich der Sattdampf wie in Fig. 11 gezeigt lediglich von außen an das Material zugeführt, kann es passieren, dass insbesondere bei einem sehr stark gepressten Ballen 72 der Sattdampf nicht ausreichend mit dem Material im Inneren des Ballens in Kontakt kommt. Statt dessen wird die Luft, die sich im Ballen befindet, durch den unter hohem Druck befindlichen Dampf im Inneren des Ballens komprimiert, möglicherweise ohne sich bei den verhältnismäßig kurzen Behandlungszeiten ausreichend mit dem heißen Dampf zu durchmischen. Durch die Verwendung des Spießes 124 wird eine gründliche Sattdampfbehandlung auch im Inneren des Ballens 72 gewährleistet.

Schließlich zeigt Fig. 14 eine weitere Einrichtung 130 zur Sattdampfbehandlung, die fünf Druckbehälter 84 umfasst, die ähnlich wie bei der Einrichtung 122 von Fig. 13 mit Spießen 124 versehen sind. Allerdings sind bei der Einrichtung 130 von Fig. 14 die Druckbehälter 84 vertikal angeordnet, so dass die Strohballen von oben mit einem Kran 132 in die Druckbehälter eingeführt werden können 84. Der Kran 132 umfasst eine Laufkatze 134 und ein Gestell 136, an dem ein oberer Greifer 138 für einen oberen Ballen und ein unterer Greifer 140 für einen unteren Ballen ausgebildet sind. Mit dem Kran 130 können somit zwei vertikal übereinander angeordnete Strohballen 72 gegriffen werden, von oben in den Druckbehälter 84 eingeführt und auf einen Spieß 124 aufgespießt werden, der zu diesem Zwecke etwa doppelt so lang ausgebildet ist wie der Spieß 124 von Fig. 13.

Sämtliche Druckbehälter 84 von Fig. 14 sind über eine Rohrleitung mit demselben Druckreservoir 112 verbunden. Dabei wird ähnlich wie in Fig. 13 der Sattdampf 102 jeweils über die Zuleitung 92 durch den Spieß 124 und durch die Strohballen 72 hindurch in den Druckbehälter 84 eingeführt.

Die Weiterbildung von Fig. 14 ist für eine Anlage mit hohem Durchsatz konzipiert, in der die Sattdampfbehandlung sehr effizient durchgeführt werden kann.

### 3.4 Einweichung in Perkolat oder ähnlichem

In dem vierten oben genannten Verfahrensschritt werden die vorbehandelten Ballen in Perkolat eingeweicht, welches eine schwach saure Lösung darstellt. Alternativ können die Ballen jedoch auch in einer schwach alkalischen Lösung, wie beispielsweise Natronlauge, eingeweicht werden. Nach dem Einweichen werden die Ballen auf rund 40°C erwärmt, was beispielsweise dadurch bewerkstelligt werden kann, dass der Stroh-Kanal 46 (s. Fig. 8) durch Abwärme der Gas-Ottomotoren geheizt wird. Durch das Einweichen des Materials nach der Sattdampfbehandlung und vor der anaeroben bakteriellen Fermentation wird eine schwach aerobe Vorhydrolyse initiiert, durch die die anschließende anaerobe bakterielle Fermentation nochmals beschleunigt wird. Bei der Einweichung mit Perkolat sind die anaeroben Bakterien gleich am Ort frischen Materials, was ebenfalls vorteilhaft ist.

Es ist wichtig, festzuhalten, dass mit dem hier beschriebenen Verfahren zum Aufschluss von Stroh, erreicht wird, dass das vorbehandelte Material in den Fennentern einen erheblichen Gasertrag bei moderaten Verweilzeiten erbringt, und dies, ohne zusätzliche Enzyme, Pilze oder Hefen zuzufügen. Der vorhandene natürliche Säuregehalt des Strohs (rund 3 bis 4 %) löst auch so die feste Cellulose auf und überführt sie in eine wässrige Lösung (Autohydrolyse). Die biogenen Polymere werden durch die Einwirkung der organischen Säure chemisch und/oder durch Einfluss von Bakterien biochemisch in nieder-molekulare Verbindungen (Monosacharide, Aminosäuren, kurzkettige Peptide, langkettige Fettsäuren, Glyzerin) gespalten. Diese liegen am Ende der Phase in wassergelöster Form vor. Dies geschieht jedoch ohne dass zunächst Enzyme, Bakterien oder Hefen zugegeben werden müssen. Das ligninhaltige Material wird in dieser Ausführungsform allein der Autohydrolyse und der bakteriellen Hydrolyse überlassen.

Der hier im Detail beschriebene Aufschluss mit den genannten vier Verfahrensschritten ist äußerst effektiv und vorteilhaft, jedoch ist es nicht zwingend notwendig, dass sämtliche vier Schritte zur Anwendung kommen, und es können auch einfachere Verfahren mit weniger bzw. nur einer Auswahl der Schritte durchgeführt werden, die immer noch eine Vergärung von ligninhaltigen nachwachsenden Rohstoffen erlauben. Insbesondere kann bereits dann ein brauchbarer Aufschluss des ligninhaltigen Materials erhalten werden, wenn dieses vor der Einbringung in den Fermenter nur mit Festmist und/oder Gülle vermischt wird, weil durch den darin enthaltenen Harnstoff die Ligninstrukturen bereits aufweichen können. Es ist dabei sogar nicht unbedingt nötig, dass der LHNawaRo vor dem Einbringen in den Fermenter mit Gülle oder Festmist vermischt wird, sondern es kann bereits ausreichen, wenn LHNawaRos und Festmist in abwechselnden Lagen im Fermenter übereinandergeschichtet werden, gegebenenfalls mit dazwischen liegenden Schichten aus anderen, nicht lignifizierten NawaRos, wobei der Harnstoff der oben liegenden Festmistschichten mit dem Perkolat in die Schicht mit dem ligninhaltigen Material dringt und die flächigen Ligninstrukturen so zumindest teilweise auflöst. Dies stellt einen sehr einfachen Fall eines chemischen Aufschlusses dar.

### Bezugszeichenliste

- 10: Biomassekraftwerk
- 12: Grundabschnitt
- 14: Ausbauabschnitt
- 16: Fermenter
- 18: Fermentervorplatz
- 20: Fermentertor
- 22: Strom- und Wärmeerzeugungsanlage
- 24: Anliefer- und Verladebereich
- 26: Fermentervorplatz-Hallenabschnitt
- 28: Anliefer- und Verladebereichshallenabschnitt
- 30: Technikbrücke
- 31: Abgas-Abkühlraum
- 32: Folien-Gasspeicher
- 34: Perkolatumlauftank
- 36: Südlicher Technikraum
- 38: Nördlicher Technikraum
- 40: Lichtbänder
- 42: Anlieferbunker für Frischmasse
- 44: Förderband
- 46: Frischmassebunker
- 48: Bereitstellungsraum
- 50: Ballen-Anlieferungsraum
- 52: Aufschlußbereich
- 54: Zwischenlager
- 56: Rollenförderer
- 58: Strohkanal
- 60: Gärrestebunker
- 62: Schütttrog für Gärreste
- 64: Weiche für Gärreste
- 66: Förderband für Gärreste
- 68: Verladesilos für Gärreste
- 70: Dehydrierungseinrichtung
- 72: Strohballen
- 74: Unterseite des Strohballens 72
- 76: Oberseite des Strohballens 72
- 78: erster Satz von Löchern
- 80: zweiter Satz von Löchern
- 82: Einrichtung zur Sattdampfbehandlung
- 83: Deckel
- 84: Druckbehälter
- 85: Scharnier
- 86: Zentraler Gasverteilspeicher
- 87: Zuführeinrichtung
- 88: Motoraufstellraum
- 89: Behälter
- 90: Zuluft Motoraufstellräume
- 91: Schließmechanismus
- 92: Entlüftung Motoraufstellräume
- 93: Zufuhrleitung
- 94: Docking Station
- 95: Zuführungsventil
- 96: Lager
- 97: Auslaßleitung
- 98: Auslaß-Ventil
- 100: Kompressor
- 102: Sattdampf
- 103: Austretender Sattdampf
- 104: Einrichtung zur Sattdampfbehandlung
- 106: ligninhaltiger nachwachsender Rohstoff
- 108: druckfester Schieber
- 110: Trichter
- 112: Dampfreservoir
- 114: Heizung
- 116: Förderband
- 117: Slurry
- 118: Rohrverbindung
- 120: Leitung zum PUT
- 122: Einrichtung zur Sattdampfbehandlung
- 124: Spieß
- 126: innerer Hohlraum
- 128: Öffnung im Spieß 124
- 130: Einrichtung zur Sattdampfbehandlung
- 132: Kran
- 134: Laufkatze
- 136: Gestell
- 138: Greifer für oberen Ballen
- 140: Greifer für unteren Ballen

## Patentansprüche

1. Biogasanlage (10) zur Erzeugung von Biogas, umfassend:
mindestens einen Fermenter für eine anaerobe bakterielle Vergärung von Biomasse nach dem Feststoffvergärungsverfahren, und
eine Einrichtung zum thermischen Aufschluss von Strohballen (82, 104, 122, 130) durch Sattdampfbehandlung oder Thermodruckhydrolyse, bei der die Einrichtung (82, 122, 130) zum thermischen Aufschluss für die Behandlung von Strohballen (72) ausgelegt ist und mindestens einen Druckbehälter (84) mit Zuführleitung (93) und Ventil (95) und Mittel (112, 114) umfasst, die ausgelegt sind, Wasserdampf mit einem Druck, der zwischen 20 und 30 bar liegt und einer Temperatur, die zwischen 180°C und 250°C liegt, zu erzeugen.

2. Biogasanlage nach Anspruch 1, weiter aufweisend eine Einrichtung zum chemischen Aufschluss von Stroh, umfassend einen Behälter zum Einweichen des Strohs in Wasser, in einer Wasser-Säure-Lösung, in einer Wasser-Laugen-Lösung, in Perkolat oder in Gülle.

3. Biogasanlage (10) nach Anspruch 1, welche zur Erzeugung von Biogas nach dem Feststoffvergärungsverfahren ausgelegt ist und eine Mehrzahl von Fermentern (16) umfasst.

4. Biogasanlage (10) nach einem der Ansprüche 1 bis 3,bei der die Sattdampfbehandlung 5 bis 15 Minuten dauert oder die Thermodruckhydrolyse 60 bis 120 Minuten dauert.

5. Biogasanlage (10) nach einem der Ansprüche 1 bis 4, bei der die Einrichtung (130) zum thermischen Aufschluss mehrere Druckbehälter (84) umfasst, die durch Rohrleitungen miteinander verbunden sind.

6. Biogasanlage (10) nach einem der Ansprüche 1 bis 5, bei der die Einrichtung (122, 130) zum thermischen Aufschluss mindestens einen Spieß (124) umfasst, auf den ein Strohballen (72) aufspiessbar ist, wobei der Spieß (124) einen inneren Hohlraum (126) aufweist, in den Wasserdampf einleitbar ist, und eine Mehrzahl von Öffnungen (128), durch die der Wasserdampf aus dem Hohlraum (126) austreten kann.

7. Biogasanlage (10) nach einem der vorhergehenden Ansprüche, mit Mitteln zum Perforieren von Strohballen (72).

8. Biogasanlage (10) nach Anspruch 7, bei der die Mittel zum Perforieren geeignet sind, einen Ballen (72) von zwei Seiten so zu perforieren, dass die bei der Perforation von einer Seite (74) entstehenden Löcher (78) und Löcher (80), die bei der Perforation von der anderen Seite (76) entstehen, durch Materialbrücken getrennt sind.

9. Biogasanlage (10) nach einem der vorhergehenden Ansprüche, bei der die Einrichtungen zum thermischen Aufschluss und ggf. chemischen Aufschluss von Stroh in einem Anliefer- und Verladebereich (24) untergebracht sind, wobei vorzugsweise der Anliefer- und Verladebereich (24) stationäre Fördertechnik (44, 46, 56) umfasst, die geeignet ist, Frischmasse aus dem Anliefer- und Verladebereich (24) zu einem Fermentervorplatz (28) zu fördern, von dem aus eine Mehrzahl von Fermentern (16) des Garagentyps zugänglich sind.

10. Biogasanlage (10) nach Anspruch 9 , welche eine Entladungsstelle für Strohballen umfasst, in der ein Kran (132) vorgesehen ist, der geeignet ist, die Ballen zu greifen und zu befördern.

11. Biogasanlage (10) nach einem der Ansprüche 1 bis 10, bei der am Austrittsende eines Gärrestebunkers (60), der vom Fermentervorplatz (18) aus zum Einbringen der Gärreste zugänglich ist, eine Einrichtung (70) zum Dehydrieren der Gärreste vorgesehen ist.

12. Biogasanlage (10) nach einem der vorhergehenden Ansprüche mit einer Trocknungsanlage zum Trocknen von Gärresten.

13. Biogasanlage (10) nach einem der vorhergehenden Ansprüche mit einer Vergasungsanlage, die geeignet ist, aus getrockneten Gärresten nach dem Verfahren der Holzvergasung Holz- bzw. Schwachgas zu erzeugen.

14. Verfahren zur Erzeugung von Biogas aus Stroh, mit den folgenden Schritten:
Vorbehandlung von ballenförmigem Stroh, um einen thermischen Aufschluss desselben durch Sattdampfbehandlung oder Thermodruckhydrolyse zu bewirken mittels einer Einrichtung (82, 104, 122, 130), die zum thermischen Aufschluss für die Behandlung von Strohballen (72) ausgelegt ist und mindestens einen Druckbehälter (84) mit Zuführleitung (93) und Ventil (95) und Mittel (112, 114) umfasst, welche ausgelegt sind, die Sattdampfbehandlung bei einer Temperatur zwischen 180°C und 240°C und einem Druck zwischen 20 und 30 bar für weniger als 20 Minuten durchzuführen oder die Thermodruckhydrolyse bei einer Temperatur zwischen 180°C und 240°C und einem Druck zwischen 20 und 30 bar für 60 bis 120 Minuten durchzuführen;
Einbringen des vorbehandelten Strohs in einen Fermenter (16) und
Erzeugen von Bedingungen im Fermenter (16), die eine anaerobe bakterielle Vergärung nach dem Feststoffvergärungsverfahren erlauben.

15. Verfahren nach Anspruch 14, bei dem zusätzlich ein chemischer Aufschluss des Strohs durch Einweichen des Strohs in Wasser, in einer Wasser-Säure-Lösung, in einer Wasser-Laugen-Lösung, in Perkolat oder in Gülle erfolgt.

16. Verfahren nach Anspruch 14, bei dem der chemische Aufschluss durch eine der Gärmasseneinbringung in den Fermenter vorgeschalteten Vermischung des Strohs mit Festmist, Gülle, Perkolat und/oder perkolatenthaltender Gärmasse geschieht.

17. Verfahren nach einem der Ansprüche 14 bis 16, bei dem die Sattdampfbehandlung so durchgeführt wird, dass er Ligninstrukturen des Strohs aufweicht, die äußere Struktur des Strohs insgesamt aber im Wesentlichen bestehen bleibt.

18. Verfahren nach einem der Ansprüche 14 bis 17, bei dem die Ballen (72) von mindestens einer Seite (74, 76) gelocht werden, wobei vorzugsweise die Löcher (78, 80) nicht vollständig durch den Ballen (72) hindurchgehen.

19. Verfahren nach einem der Ansprüche 14 bis 18, bei dem die Ballen (72) für die Sattdampfbehandlung auf mindestens einen Spieß (124) aufgespießt werden und der Dampf durch Öffnungen (128) in dem Spieß (124) in das Innere des Ballens (72) eingeführt wird.

20. Verfahren nach einem der Ansprüche 14 bis 19, bei dem der Behandlungsdruck am Ende der Sattdampfbehandlung innerhalb von fünf Sekunden um mindestens 80% gesenkt wird.

21. Verfahren nach einem der Ansprüche 14 bis 20, bei dem Gärreste dehydriert werden.

22. Verfahren nach einem der Ansprüche 14 bis 21, bei dem Gärreste auf einen Wasseranteil von unter 25%, vorzugsweise unter 15%, getrocknet werden und/oder bei dem Gärreste zu Holz- oder Schwachgas vergast werden.

## Claims

1. A biogas plant (10) for the production of biogas, comprising:
at least one fermenter for the anaerobic bacterial fermentation of biomass according to the solid fermentation method, and
a device for the thermal decomposition of straw bales (82, 104, 122, 130) by saturated steam treatment or by thermal pressure hydrolysis, wherein said device (82, 122, 130) is construed for the thermal decomposition for the treatment of straw bales and comprises at least a pressure container (84) with a feeding line (93) and valve (95) and means (112, 114) which are suitable to produce steam at a pressure which is between 20 and 30 bar and a temperature which is between 180°C and 250°C.

2. The biogas plant according to claim 1, further including a device for the chemical break down of straw, comprising a container for soaking the straw in water, in a water-acid solution, in a water-lye solution, in percolate or in liquid manure.

3. The biogas plant (10) according to claim 1, which is designed for producing-biogas according to the solid fermentation method and which comprises a plurality of fermenters (16).

4. The biogas plant (10) according to any of claims 1 to 3, wherein the saturated steam treatment takes 5 to 15 minutes or the thermal pressure hydrolysis takes 60 to 120 minutes.

5. The biogas plant (10) according to any of claims 1 to 4, wherein the device (130) for the thermal decomposition comprises a plurality of pressure containers (84) which are connected to one another by conduits.

6. The biogas plant (10) according to any of claims 1 to 5, wherein the device (122, 130) for the thermal decomposition comprises at least a skewer (124) for spiking a straw bale (72), wherein the skewer (124) has an internal hollow space (126) where steam can be induced, and a plurality of openings (128) through which the steam can exit the hollow space (126).

7. The biogas plant (10) according to any of the preceding claims, having means for perforating of straw bales (72).

8. The biogas plant (10) according to claim 7, wherein the means for perforating are suitable to perforate a straw bale (72) at two sides so that the holes (78) which result from perforating the one side (74) and the holes (80) which result from perforating the other side (76) are separated by material bridges.

9. The biogas plant (10) according to any of the preceding claims, wherein, for the thermal decomposition and optionally for the chemical decomposition of straw, the devices are accommodated in a delivery and loading area (24), wherein the delivery and loading area (24) preferably comprises stationary conveyance technology (44, 46, 56), which is suitable to convey fresh mass from the delivery and loading area (24) to a fermenter forecourt (28) from where a plurality of garage-type fermenters (16) can be accessed.

10. The biogas plant (10) according to claim 9, having an unloading site for straw bales, wherein a crane (132) is provided at the unloading site and is suitable to grip and convey straw bales.

11. The biogas plant (10) according to any of claims 1 to 10, in which a device (70) for the dehydration of the digestate is provided at the outlet end of a digestate bunker (60) which is accessible from the fermenter forecourt (18) for loading the digestate.

12. The biogas plant (10) according to any of the preceding claims, comprising a drying system for drying digestate.

13. The biogas plant (10) according to any of the preceding claims having a gasification plant which is suitable to provide, according to the process of wood distillation, wood and lean gas, respectively, from the dried digestate.

14. A method for producing biogas from straw, comprising the following steps:
pretreating bale-shaped straw to effect a thermal decomposition by saturation steam treatment or by thermal pressure hydrolysis using a device (82, 104, 122, 130) that is construed for the thermal decomposition for the treatment of straw bales (72) and comprises at least a pressure container (84) with a feeding line (93) and valve (95) and means (112, 114) which are suitable to conduct saturation steam treatment at a temperature between 180° C and 240°C and a pressure between 20 and 30 bar for less than 20 minutes or a thermal pressure hydrolysis treatment at a temperature between 180°C and 240°C and a pressure between 20 and 30 bar for 60 to 120 minutes;
introducing the pretreated straw into a fermenter (16) and
producing conditions in the fermenter (16) which permit an anaerobic bacterial fermentation according to the solid fermentation method.

15. The method according to claim 14, wherein additionally a chemical decomposition of the straw is effected by soaking the straw in water, in a water-acid solution, in a water-lye solution, in percolate or in liquid manure.

16. The method according to claim 14, wherein the chemical decomposition is conducted by mixing the straw with solid manure, liquid manure, percolate and/or percolate-containing fermentation mass prior to introducing of the fermentation mass into the fermenter.

17. The method according to any of claims 14 to 16, wherein the saturation steam treatment is carried out in such a way that it macerates the lignin structures of the straw while the outer structure of the straw is substantially maintained on the whole.

18. The method according to any of claims 14 to 17, wherein the bales (72) are spiked from at least one side (74,76), wherein preferably the holes (78, 80) do not completely extend through the bale (72).

19. The method according to any of claims 14 to 18, wherein the bales (72) are spiked at least at a skewer (124) for the saturated steam treatment and the steam is introduced into the internal part of the bale (72) through openings (128) in the skewer (124).

20. The method according to any of claims 14 to 19, wherein the treatment pressure is lowered at the end of the saturation steam treatment by at least 80 % within five seconds.

21. The method according to any of claims 14 to 20, wherein the digestate is dehydrated.

22. The method according to any of claims 14 to 21, wherein digestate is dried to a water content of below 25 %, preferably below 15 %, and gasified into wood and/or lean gas.

## Revendications

1. Installation de biogaz (10) pour la production de biogaz, comprenant:
au moins un fermenteur destiné à une fermentation bactérienne anaérobie de biomasse selon le procédé de fermentation de matières solides, et
un dispositif de désintégration thermique de balles de paille (82, 104, 122, 130) par traitement à la vapeur saturée ou par hydrolyse par transfert thermique, où le dispositif (82, 122, 130) est conçu pour la désintégration thermique pour le traitement de balles de paille (72) et comporte au moins un récipient sous pression (84) avec un conduit d'alimentation (93) et une soupape (95) et des moyens (112, 114) qui sont conçus pour générer de la vapeur d'eau à une pression comprise entre 20 et 30 bars et à une température comprise entre 180°C et 250°C.

2. Installation de biogaz selon la revendication 1, présentant par ailleurs un dispositif pour la désintégration chimique de la paille, comprenant un récipient pour le trempage de la paille dans de l'eau, dans une solution d'eau et d'acide, dans une solution d'eau et de soude, dans du percolé ou dans du lisier.

3. Installation de biogaz (10) selon la revendication 1, qui est conçue pour la production de biogaz selon le procédé de fermentation de matières solides et comporte une pluralité de fermenteurs (16).

4. Installation de biogaz (10) selon l'une des revendications 1 à 3, dans laquelle le traitement à la vapeur saturée dure de 5 à 15 minutes ou l'hydrolyse par transfert thermique dure de 60 à 120 minutes.

5. Installation de biogaz (10) selon l'une des revendications 1 à 4, dans laquelle le dispositif (130) de désintégration thermique comporte plusieurs récipients sous pression (84) qui sont connectés l'un à l'autre par des tuyauteries.

6. Installation de biogaz (10) selon l'une des revendications 1 à 5, dans laquelle le dispositif (122, 130) de désintégration thermique comporte au moins une broche (124) sur laquelle peut être empalée une balle de paille (72), dans laquelle la broche (124) présente une cavité intérieure (126) dans laquelle peut être introduite de la vapeur d'eau, et une pluralité d'ouvertures (128) à travers lesquelles la vapeur d'eau peut sortir de la cavité (126).

7. Installation de biogaz (10) selon l'une des revendications précédentes, avec des moyens pour perforer les balles de paille (72).

8. Installation de biogaz (10) selon la revendication 7, dans laquelle les moyens de perforation conviennent pour perforer une balle (72) de deux côtés de sorte que les trous (78) se formant lors de la perforation d'un côté (74) et les trous (80) se formant lors de la perforation de l'autre côté (76) soient séparés par des ponts de matière.

9. Installation de biogaz (10) selon l'une des revendications précédentes, dans laquelle les moyens de désintégration thermique et éventuellement de désintégration chimique de la paille sont logés dans une zone de livraison et de chargement (24), la zone de livraison et de chargement (24) comportant de préférence une technique de transport stationnaire (44, 46, 56) qui convient pour transporter de la masse fraîche de la zone de livraison et de chargement (24) vers un parvis de fermenteur (28) à partir duquel sont accessibles une pluralité de fermenteurs (16) de type garage.

10. Installation de biogaz (10) selon la revendication 9, qui comporte un point de déchargement pour balles de paille auquel est prévue une grue (132) qui convient pour saisir et transporter les balles de paille.

11. Installation de biogaz (10) selon l'une des revendications 1 à 10, dans laquelle, à l'extrémité de sortie d'une trémie à résidus de fermentation (60) qui est accessible depuis le parvis du fermenteur (18) pour l'introduction de résidus de fermentation, est prévu un dispositif (70) de déshydratation des résidus de fermentation.

12. Installation de biogaz (10) selon l'une des revendications précédentes, avec une installation de séchage pour le séchage de résidus de fermentation.

13. Installation de biogaz (10) selon l'une des revendications précédentes, avec une installation de gazéification qui convient pour générer du gaz de bois ou du gaz pauvre à partir de résidus de fermentation séchés par le procédé de gazéification de bois.

14. Procédé de production de biogaz à partir de paille, aux étapes suivantes consistant à:
prétraiter la paille en forme de balle pour provoquer une désintégration thermique de cette dernière par traitement à la vapeur saturée ou hydrolyse par transfert thermique au moyen d'un dispositif (82, 104, 122, 130) qui est conçu pour la désintégration thermique pour le traitement de balles de paille (72) et comporte au moins un récipient sous pression (84) avec un conduit d'alimentation (93) et une soupape (95) et des moyens (112, 114) qui sont conçus pour effectuer le traitement à la vapeur saturée à une température comprise entre 180°C et 240°C et à une pression comprise entre 20 et 30 bars et une pression comprise entre 20 et 30 bars pendant moins de 20 minutes ou pour effectuer l'hydrolyse par transfert thermique à une température comprise entre 180°C et 240°C et une pression comprise entre 20 à 30 bars pendant de 60 à 120 minutes;
introduire la paille prétraitée dans un fermenteur (16), et
générer dans le fermenteur (16) des conditions qui permettent une fermentation bactérienne anaérobie selon le procédé de fermentation de matières solides.

15. Procédé selon la revendication 14, dans lequel a lieu en outre une désintégration chimique de la paille par trempage de la paille dans de l'eau, dans une solution d'eau et d'acide, dans une solution d'eau et de soude, dans du percolé ou dans du lisier.

16. Procédé selon la revendication 14, dans lequel la désintégration chimique a lieu par un mélange, connecté avant l'introduction de masse de fermentation dans le fermenteur, de la paille avec du fumier solide, du lisier, du percolé et/ou de la masse de fermentation contenant du percolé.

17. Procédé selon l'une quelconque des revendications 14 à 16, dans lequel le traitement à la vapeur saturée est effectué de sorte qu'il ramollisse les structures de lignine de la paille, mais que persiste substantiellement la structure externe de la paille dans son ensemble.

18. Procédé selon l'une des revendications 14 à 17, dans lequel les balles (72) sont perforées d'au moins un côté (74, 76), dans lequel de préférence les trous (78, 80) ne traversent pas totalement la balle (72).

19. Procédé selon l'une des revendications 14 à 18, dans lequel les balles (72) sont, pour le traitement à la vapeur saturée, empalées sur au moins une broche (124) et la vapeur est introduite à travers des ouvertures (128) dans la broche (124) à l'intérieur de la balle (72).

20. Procédé selon l'une des revendications 14 à 19, dans lequel, à la fin du traitement à la vapeur saturée, la pression de traitement est réduite d'au moins 80% en l'espace de cinq secondes.

21. Procédé selon l'une des revendications 14 à 20, dans lequel les résidus de fermentation sont déshydratés.

22. Procédé selon l'une des revendications 14 à 21, dans lequel les résidus de fermentation sont séchés à une teneur en eau de moins de 25%, de préférence de moins de 15%, et/ou dans lequel les résidus de fermentation sont gazéifiés pour obtenir du gaz de bois ou du gaz pauvre.
